# EUROPÄISCHE PATENTSCHRIFT

(11) **EP 4 338 842 B1**
(45) Veröffentlichungstag und Bekanntmachung des Hinweises auf die Patenterteilung: **23.10.2024**
(21) Anmeldenummer: 22195306.0
(22) Anmeldetag: 13.09.2022
(51) Int. Cl.: B01L 3/02, B29C 45/76, B29C 45/17, A61M 5/00

(54) **VERPACKUNGSVERFAHREN FÜR PIPETTENSPITZEN ODER MEDIZINISCHE REAKTIONSGEFÄSSE MIT SEITENPRÜFUNG, VERPACKUNGSVORRICHTUNG SOWIE SPRITZGUSSSYSTEM**
PACKAGING METHOD FOR PIPETTE TIPS OR MEDICAL REACTION VESSELS WITH LATERAL TESTING, PACKAGING DEVICE AND INJECTION MOLDING SYSTEM
PROCÉDÉ D'EMBALLAGE POUR POINTES DE PIPETTES OU RÉCIPIENTS DE RÉACTION MÉDICAL AVEC CONTRÔLE LATÉRAL, DISPOSITIF D'EMBALLAGE ET SYSTÈME DE MOULAGE PAR INJECTION

(43) Veröffentlichungstag der Anmeldung: 20.03.2024
(73) Patentinhaber: Waldorf Technik GmbH, 78234 Engen (DE)
(72) Erfinder: BOOS, Christian, 79194 Gundelfingen (DE); FUHRMANN, Ralf, 78250 Tengen (DE)
(74) Vertreter: Patent- und Rechtsanwälte Behrmann Wagner PartG mbB

(56) Entgegenhaltungen:
- EP-B1- 2 323 930
- EP-B1- 2 801 531

## Beschreibung

Die Erfindung betrifft ein Verpackungsverfahren gemäß dem Oberbegriff des Anspruchs 1 zum Verpacken von als Pipettenspitzen oder medizinische Reaktionsgefäße ausgebildeten Kunststoffspritzgussteilen in Endverpackungseinheiten, wobei die Kunststoffspritzgussteile jeweils eine, insbesondere vertikal orientierte Längsmittelachse aufweisen, die ganz besonders bevorzugt eine jeweilige Öffnung, insbesondere Befüllöffnung des jeweiligen Kunststoffspritzgussteils, vorzugsweise zentrisch, durchsetzt. Besonders bevorzugt sind die Kunststoffspritzgussteile bezogen auf deren Längsmittelachse rotationssymmetrisch ausgebildet. Das Verpackungsverfahren umfasst die Schritte: Mehrfaches, jeweils gleichzeitiges Entnehmen eines jeweiligen Schusses von K Kunststoffspritzgussteilen aus K Kavitäten, wobei es bevorzugt ist, wenn die K Kavitäten, bevorzugt gleichmäßig, auf C Cluster (geometrische Kavitätenanordnungen) verteilt angeordnet sind, gleichmäßiges und kavitätenreines Verteilen der K Kunststoffspritzgussteile auf U kavitätenreine Untergruppen in einer ersten Ablageebene, wobei gemäß einer bevorzugten Ausführungsform des Verfahrens das gleichmäßige, kavitätenreine Verteilen der K Kunststoffspritzgussteilte auf die U kavitätenreinen Untergruppen in der ersten Ablageebene solange erfolgt, bis jede Untergruppe L Kunststoffspritzgussteile aufweist, wobei die Anzahl L der Kunststoffspritzgussteile einer (jeder) der Untergruppen der Anzahl A der in einer Endverpackungseinheit maximal aufnehmbaren Kunststoffspritzgussteilen oder einem ganzzahligen Teiler von A entspricht, kavitätenreines oder kavitätensortiertes Auffüllen der Endverpackungseinheiten, bis die Endverpackungseinheiten jeweils A Kunststoffspritzgusstele aufweisen.

Ferner betrifft die Erfindung eine Verpackungsvorrichtung gemäß dem Oberbegriff des Anspruchs 9 zum Zusammenwirken mit einer benachbart zur Verpackungsvorrichtung anordnenbaren Spritzgussvorrichtung, deren Spritzgusswerkzeuge ausgebildet sind zum Herstellen von Pipettenspitzen oder medizinischen Reaktionsgefäßen.

Darüber hinaus betrifft die Erfindung ein Spritzgusssystem gemäß Anspruch 15, umfassend eine Spritzgussvorrichtung sowie eine Verpackungsvorrichtung.

Bei bekannten Verpackungsverfahren zum Verpacken von als Pipettenspitzen oder medizinische Reaktionsgefäße, insbesondere zur Blutanalyse ausgebildeten Kunststoffspritzgussteilen werden gleichzeitig K Kunststoffspritzgussteile aus K Kavitäten einer Spritzgussvorrichtung entnommen und als Schüttgut zwischengespeichert. Aus dieser Schüttung werden dann Endverpackungseinheiten jeweils mit A Kunststoffspritzgussteilen beladen. Nachteilig bei dem bekannten Verfahren ist, dass keine Zuordnung der abgepackten Kunststoffteile zu bestimmten Kavitäten möglich ist. Es ist also nicht mehr nachvollziehbar, aus welcher Kavität bzw. aus welchen Kavitäten die Kunststoffspritzgussteile einer bestimmten Endverpackungseinheit stammen. Dies führt bei nur einem einzigen fehlerhaften Kunststoffspritzgussteil in einer Endverpackungseinheit dazu, dass die gesamte Produktion zurückgerufen werden muss und nicht nur die mit Kunststoffspritzgussteilen aus einer bestimmten Kavität bzw. aus bestimmten Kavitäten bestückt in Endverpackungseinheiten.

Revolutioniert wurde die Verpackung von als Pipettenspritzen oder medizinische Reaktionsgemäße ausgebildeten Kunststoffspritzgussteilen durch die in der EP 2 323 930 B1 (WO 2011/003507 A1) beschriebene Erfindung. Diese schlägt vor, dass die gleichzeitig aus den K Kavitäten mittels eines Entnahmegreifers entnommenen K Kunststoffspritzgussteile gleichzeitig auf U Untergruppen verteilt in einem Zwischenspeicher abgelegt werden, und dass mehrfach und solange K Kunststoffspritzgussteile aus den K Kavitäten entnommen und gleichzeitig auf die Untergruppen in dem Zwischenspeicher, d.h. vor dem Überführen in die Endverpackungen verteilt werden, bis jede Untergruppe L Kunststoffspritzgussteile aufweist, wobei die Anzahl L der Kunststoffspritzgussteile einer (jeder) Untergruppe der Anzahl von in einer Endverpackungseinheit maximal zu ladenden Kunststoffspritzgussteilen oder einem ganzzahligen Teil davon A entspricht. Daraufhin werden die Endverpackungseinheiten jeweils mit einer einzigen Untergruppe, also kavitätenrein befüllt oder mit mehreren dieser Untergruppen, also kavitätensortiert.

Die in der EP 2 801 531 B1 der Anmelderin beschriebene Erfindung bildet das vorgenannte Verfahren weiter, in dem sie ermöglicht, auf einen Zwischenspeicher zu verzichten und die Ablageebene, in der die kavitätenreinen Untergruppen gebildet werden, unmittelbar von den Endverpackungseinheiten bereitgestellt wird.

Bei der in der EP 2 868 587 B1 der Anmelderin beschriebenen Erfindung werden die kavitätenreinen Untergruppen auf einem Tisch einer Wechseltischanordnung gebildet und daraufhin durch Verdrehen der Wechseltischanordnung um eine Horizontalachse in vertikaler Richtung verstellt, um den zur Verfügung stehenden Raum optimal auszunutzen.

Aus der EP 2 801 532 B1 der Anmelderin ist ein weiteres, optimiertes Verfahren zum kavitätenreinen oder kavitätensortierten Verpacken von Pipettenspitzen und medizinischen Reaktionsgefäßen bekannt, bei welchem jeweils ein einziger Schuss von Spritzgussteilen vor der Bildung von kavitätenreinen Untergruppen in einen Zwischenpuffer zwischengepuffert wird, um hierdurch die Zykluszeit zu verkürzen.

Aus der DE 10 2017 118 527 A1 der Anmelderin ist ein weiter optimiertes Verpackungsverfahren für Pipettenspitzen bekannt, das das aus der EP 2 323 930 B1 der Anmelderin bekannte Grund- bzw. Basisverfahren dahingehend optimiert, dass vor der Anordnung der Kunststoffspritzgussteile in kavitätenreinen Untergruppen eine Linienbildung der Spritzgussteile eines jeweiligen Schusses erfolgt. Die linienförmig angeordneten Spritzgussteile eines Schusses werden dann unmittelbar in kavitätenreinen Untergruppen angeordnet.

In der WO 2022/022955 A1 der Anmelderin ist ein weiter optimiertes Verpackungsverfahren für Pipettenspitzen und als medizinische Reaktionsgemäße ausgebildete Kunststoffspritzteile beschrieben, mit welchem die Zykluszeit noch weiter reduziert werden kann. Der dort beschriebenen Erfindung liegt der Gedanke zugrunde nicht jeden Schuss von K Kunststoffspritzgussteilen für sich in die kavitätenreinen Untergruppen zu überführen, sondern zunächst einer der Anzahl der Kavitäten entsprechende Anzahl von kavitätenreinen Unter-Untergruppen zu bilden, die jeweils mehrere Kunststoffspritzgussteile aus derselben Kavität aufnehmen, wobei die Untergruppen dann Schritt für Schritt mit kavitätenreinen Unter-Untergruppen befüllt bzw. gebildet werden. Hierdurch kann die Verstell- bzw. Greifermechanik robuster und insbesondre biegesteifer ausgebildet werden.

Sämtliche vorgenannten Verpackungsverfahren mit zugehörigen Verpackungsvorrichtungen der Anmelderin für als Pipettenspritzen oder medizinische Reaktionsgefäße ausgebildeten Kunststoffspritzgussteilen haben sich bewährt und gewährleisten seither eine optimierte Nachverfolgbarkeit von Produkten. Auch wurde durch die beschriebenen Erfindungen die Zykluszeit verbessert.

In der Praxis werden die in kavitätenreine Untergruppen aufgeteilten Kunststoffspritzgussteile zu 100% einer optischen Prüfung von oben unterzogen, wobei hierzu die Blickachse der zum Einsatz kommenden Kamera parallel zu den Längsachsen der Kunststoffspritzgussteile, also in vertikaler Richtung orientiert und nicht auf die Mantelfläche der Kunststoffspritzgussteile ist. Bei der optischen Prüfung von oben und/oder unten kann der Durchmesser und die Konzentrizität der Kunststoffspritzgussteile überprüft werden. Ebenso können Beschädigungen im oberen oder unteren Randbereich der Kunststoffspritzgussteile festgestellt werden. Eine Seitenprüfung sämtlicher Kunststoffspritzgussteile zum Feststellen von Form- und/oder Oberflächenfehlern zum Ausschließen von sporadischen Fehlern ist bisher bei gleichzeitiger Gewährleistung einer kavitätenreinen oder kavitätensortierten Verpackung in Endverpackungen nicht möglich, jedoch aufgrund der damit verbundenen, erhöhten Produktsicherheit wünschenswert. Unter Formfehlern werden dabei beispielhaft Längenfehler und/oder Breitenfehler und/oder Symmetriefehler, insbesondere Rotationssymmetriefehler verstanden. Oberflächenfehler sind beispielswiese Verunreinigungen und/oder Einschlüsse und/oder Beschädigungen, insbesondere Kratzer und/oder Löcher.

Der Erfindung liegt daher die Aufgabe zugrunde, ein im Hinblick auf die Produktsicherheit weiter verbessertes Verpackungsverfahren anzugeben, mit welchem auch sporadische Fehler, insbesondere Form- und/oder Oberflächenfehler trotz kavitätenreiner Anordnung der als Pipettenspitzen oder medizinische Reaktionsgefäße ausgebildeten Kunststoffspritzgussteilen in Untergruppen (Feldern) möglich ist. Anders ausgedrückt soll das verbesserte Verfahren eine mantelseitige 100%-Prüfung, d.h. Prüfung sämtlicher als Pipettenspitzen oder medizinische Reaktionsgefäße ausgebildeten Kunststoffspritzgussteilen ermöglichen und dabei weiterhin eine kavitätenreine Anordnung der Kunststoffspritzgussteile in Untergruppen ermöglichen sowie eine kavitätenreine oder kavitätensortierte Befüllung der Endverpackungseinheiten.

Ferner besteht die Aufgabe darin, eine Verpackungsvorrichtung anzugeben, die zur Durchführung eines wie zuvor beschrieben optimierten Verfahrens möglich ist, ebenso wie ein entsprechend optimiertes Spritzgusssystem.

Diese Aufgabe wird hinsichtlich des Verfahrens mit den Merkmalen des Patentanspruchs 1, hinsichtlich der Vorrichtung mit den Merkmalen des Patentanspruchs 9 sowie im Hinblick auf das Spritzgusssystem mit den Merkmalen des Anspruchs 15 gelöst.

Vorteilhafte Weiterbildungen der Erfindung sind in den Unteransprüchen angegeben.

Der Erfindung liegt der Gedanke zugrunde, die, bevorzugt jeweils L, Kunststoffspritzgussteile der Untergruppen vor dem Auffüllen der Endverpackungen in einer Seitenprüfeinrichtung einer optischen Seitenprüfung auf, bevorzugt sporadische, Fehler, ganz besonders bevorzugt auf Form- und/oder Oberflächenfehler, zu unterziehen, wobei die Seitenprüfeinrichtung nicht nur eine einzige digitale Kamera sondern mehrere, insbesondere in einer Umfangsrichtung um eine Prüfposition für Kunststoffspritzgussteile herum angeordnete, insbesondere gleichmäßig verteilt angeordnete digitale Kameras zum Zusammenwirken mit einer Prüflogik umfasst. Damit eine 100%-Seitenprüfung, d.h. eine Prüfung sämtlicher zuvor zu kavitätenreinen Untergruppen (Feldern) zusammengefassten bzw. sortierten, als Pipettenspitzen oder medizinische Reaktionsgefäße ausgebildeten Kunststoffspritzgussteilen möglich ist, ist erfindungsgemäß vorgesehen, die kavitätenreinen Untergruppen in jeweils S, bevorzugt kavitätenreine, ganz besonders bevorzugt gleich große, Subgruppen mit, insbesondere linienförmig, hintereinander angeordneten Kunststoffspritzgussteilen aufzuteilen und die S Subgruppen nacheinander, jeweils entlang ihrer Längs- bzw. Linienerstreckung, mit Transfermitteln durch die Seitenprüfeinrichtung zu fördern und dort der Seitenprüfung zu unterziehen. Bevorzugt werden die hintereinander angeordneten Kunststoffspritzgussteile dabei nacheinander durch eine Prüfposition bewegt, an der sie von der mindestens einen Digitalkamera mantelseitig betrachtet bzw. aufgenommen werden. Um die Kunststoffspritzgussteile über ihren gesamten Umfang bzw. mantelseitig zu prüfen ist es, wie erwähnt, bevorzugt, mehrere, in einer Umfangsrichtung, insbesondere um die vorerwähnte Prüfposition herum angeordnete, weiter bevorzugt gleichverteilte Digitalkameras vorzusehen. Bevorzugt befindet sich je mindestens eine Digitalkamera zu beiden Seiten einer Transfer- bzw. Transportachse der Subgruppen durch die Seitenprüfeinrichtung. Durch die Vereinzelung der Untergruppen in Subgruppen, insbesondere in Form von linienförmig hintereinander angeordneten Kunststoffspritzgussteilen wird die Möglichkeit geschaffen, sämtliche Kunststoffspritzgussteile einer Untergruppe nacheinander einer optischen Seitenprüfung auf sporadische Fehler, insbesondere Form- und/oder Oberflächenfehler zu unterziehen.

Erfindungsgemäß vorgesehen, dass die S Subgruppen nach dem Durchlaufen der Seitenprüfung (erneut) zu kavitätenreinen Untergruppen angeordnet werden, und zwar in einer zweiten, bevorzugt horizontalen, Ablageebene. Dabei bleibt gemäß einer bevorzugten Ausführungsform die Zusammensetzung der Untergruppen gleich - anders ausgedrückt entspricht bevorzugt die Zusammensetzung der geprüften Untergruppen in der zweiten Ablageebene, zumindest bei erfolgreicher optischer Prüfung, d.h. dann, wenn keine oder noch akzeptable Fehler, insbesondere Form- und/oder Oberflächenfehler bei Kunststoffspritzgussteilen in der jeweiligen Untergruppe festgestellt und deshalb keine Kunststoffspritzgussteile dieser Untergruppe ausgetauscht wurden, der Zusammensetzung der (entsprechenden) Untergruppen in der ersten, bevorzugten horizontalen, Ablageebene. Anders ausgedrückt wird vorzugsweise die Zusammensetzung der Untergruppen durch die Funktion des ersten und zweiten Subgruppengreifers nicht verändert, sondern nur für den Fall, dass fehlerhafte Kunststoffspritzgussteile gegen fehlerfreie Kunststoffspritzgussteile aus der jeweils selben Kavität ausgetauscht wurden. Für die nicht aussortierten Kunststoffspritzgussteile bleibt also der Untergruppenbezug vor und nach der Prüfung bevorzugt gleich. Anstatt einzelne fehlerhafte Kunststoffspritzgussteile auszutauschen ist es auch denkbar, die gesamte Untergruppe auszusortieren bzw. zu verwerfen. Grundsätzlich ist das Austauschen einzelner Kunststoffspritzgussteile oder Verwerfen ganzer Untergruppen fakultativ und nicht obligatorischer, jedoch bevorzugter, Bestandteil des erfindungsgemäßen Verfahrens sowie der erfindungsgemäßen Vorrichtung.

In Weiterbildung der Erfindung kann vorgesehen werden, dass als fehlerhaft identifizierte Kunststoffspritzgussteile vor der Bildung der geprüften, kavitätenreinen Untergruppen in der zweiten Ablageebene aussortiert und durch passende, d.h. kavitätenreine Kunststoffspritzgussteile ersetzt werden. Alternativ ist es denkbar, auch die fehlerbehafteten Kunststoffspritzgussteile zunächst in geprüfte, kavitätenreine Untergruppen in der zweiten Ablageebene anzuordnen und in einem nachfolgenden Schritt durch kavitätenreine Kunststoffspritzgussteile auszutauschen. Auch ist es wie erwähnt denkbar, bei erkennen eines fehlerhaften Teiles in einer Untergruppe, die gesamte Untergruppe auszusortieren.

Im Rahmen der Erfindung ist vorgesehen, dass die zweite Ablageebene entweder unmittelbar von den Endverpackungen gebildet wird oder von einem statischen oder verstellbaren, bevorzugt horizontalen, Zwischenspeicher, aus dem die geprüften, kavitätenreinen Untergruppen dann in die Endverpackungseinheiten überführt werden, entweder eine einzige geprüfte Untergruppe pro Endverpackungseinheit (kavitätenrein) oder mehrere (nicht sämtliche) kavitätenreine Untergruppen pro Endverpackungseinheit (kavitätensortiert).

Wie erwähnt bleibt die Zusammensetzung der geprüften, kavitätenreinen Untergruppen in der zweiten Ablageebene bevorzugt gleich wie die Zusammensetzung der jeweils entsprechenden Untergruppe in der ersten Ablageebene, also von dem Aufteilen der Untergruppen in Subgruppen, Fördern der Subgruppen durch die Seitenprüfeinrichtung und dann rekombinieren der geprüften Subgruppen in der zweiten Ablageebene unbeeinflusst. Eine (geringfügig) unterschiedliche Zusammensetzung der noch nicht geprüften und geprüften Untergruppen kann vorzugsweise nur aus dem fakultativen Aussortieren von als mittels der Seitenprüfeinrichtung und/oder mindestens einer fakultativen weiteren Prüfeinrichtung (z.B. einer vorerwähnte Prüfung von oben und/oder unten) als Fehlerhaft erkannten Kunststoffspritzgussteilen resultieren. Für den Fall der Nicht-Aussortierung einzelner Kunststoffspritzgussteile bleibt die Zusammensetzung der Untergruppen vor und nach der Prüfung also vorzugsweise gleich. Dabei ist es denkbar, die, bevorzugt von jeweils einer Subgruppe gebildeten Reihen der Kunststoffspritzgussteile in der jeweils geprüften Untergruppe identisch relativ zueinander anzuordnen wie in der zugehörigen Untergruppe in der ersten Ablageebene oder anders, beispielsweise in umgekehrter Reihenfolge, solange die Zusammensetzung insgesamt bei erfolgreicher Prüfung, d.h. bei Nichtfeststellen eines Fehlers gleichbleibt. Bevorzugt sind sowohl die Untergruppen in der ersten Ablageebene als auch die geprüften Untergruppen in der zweiten Ablageebene geometrisch als Felder mit einer bevorzugt rechteckigen Hüllkontur in der jeweiligen Ablageebene angeordnet. Die Hüllkontur ist dabei nicht auf eine Rechteckform begrenzt. Grundsätzlich sind alle aus Linien bzw. Reihenanordnungen von Kunststoffspritzgussteilen zusammensetzbare Feld- bzw. Hüllkonturformen realisierbar, so zum Beispiel auch sechs- oder achteckige Hüllkonturen, etc. Für den Fall, dass der Untergruppenbezug nicht gleich bleibt, können die Subgruppen in der zweiten Ablageebene zu neuen Untergruppen formiert werden, solange diese weiterhin kavitätenrein sind.

Im Hinblick auf die Bildung/Schaffung der Untergruppen in der ersten Ablageebene gibt es unterschiedliche Möglichkeiten - hierzu wird ausdrücklich auf, insbesondere die in der Beschreibungseinleitung erwähnten, früheren Patentanmeldungen und Patente der Anmelderin verwiesen. Denkbar ist es beispielsweise die Untergruppen nach einem jeweiligen Schuss um immer ein einziges Kunststoffspritzgussteil zu erweitern oder alternativ zunächst kavitätenreine Unter-Untergruppe zu bilden und dann die Untergruppen in der ersten Ablageebene aus kavitätenreinen Unter-Untergruppen zusammenzusetzen.

Unabhängig von der Art und Weise der Bildung der kavitätenreinen Untergruppen in der ersten Ablageebene ist es bevorzugt, wenn diese kavitätenreinen Untergruppen (bevorzugt ebenso wie die geprüften Untergruppen) als Felder mit einer rechteckigen Hüllkontur ausgebildet werden. Dabei ist es besonders bevorzugt, wenn die Untergruppen aus nebeneinander angeordneten, parallelen Linien von Kunststoffspritzgussteilen gebildet werden/sind, wobei es besonders bevorzugt ist, wenn jede zu prüfende Subgruppe von einer dieser Linien, jeweils umfasst mehrere, insbesondre S, Kunststoffspritzgussteilen gebildet wird/ist.

Besonders bevorzugt ist eine Ausführungsvariante des Verfahrens, bei welcher die Untergruppen in der ersten Ablageebene zunächst fertiggestellt, d.h. komplettiert werden, bevor eine Aufteilung in die S Subgruppen erfolgt. Von den Patentansprüchen bevorzugt erfasst sein soll auch eine alternative Ausführungsform, bei der bereits mit der Subgruppenbildung bzw. Subgruppenentnahme zu Seitenprüfzwecken begonnen wird, bevor die Untergruppen in der ersten Ablageebene komplettiert sind.

Im Hinblick auf die Anordnung der mindesten einen Digitalkamera ist vorgesehen, dass diese auf die Seitenfläche/Mantelfläche des jeweils zu prüfenden Kunststoffspritzgussteils gerichtet ist. Besonders bevorzugt ist es dabei, wenn eine zentrisch durch die Linsenanordnung der Kamera verlaufende optische (Kamera-)Achse bzw. Kameranulllinie die Längsmittelachse des zu prüfenden Kunststoffspritzgussteils winklig schneidet. Besonders zweckmäßig ist es, wie erwähnt, zur Abdeckung als besonders großen Mantelflächenbereichs mehrere, Digitalkameras, insbesondere vier, ganz besonders bevorzugt gleichmäßige, in einer Umfangsrichtung beabstandete Digitalkameras vorzusehen, insbesondere mindestens eine Kamera, bevorzugt zwei Kameras, auf jeder Seite einer später noch zu erläuternden Prüfspur bzw. auf beiden Seiten einer Transferachse. Die optische Kamerachse der mindestens einen digitalen Kamera verläuft bevorzugt winklig zur Vertikalen und liegt beispielsweise in der Horizontalen.

Wie erwähnt ist es bevorzugt, wenn die Kunststoffspritzgussteile jeder Subgruppe, bevorzugt linienförmig, hintereinander angeordnet sind, bevorzugt entlang einer Transferachse, entlang derer sie durch die Prüfeinrichtung bewegt werden.

Die Digitalkameras wirken mit einer Prüflogik zusammen, die, insbesondere mittels Bildverarbeitungssoftware, bevorzugt sporadische, Fehler erkennt, insbesondere Form- und/oder Oberflächenfehler, wobei in Weiterbildung der Erfindung vorgesehen ist, dass bei Erkennen von nicht akzeptablen Fehlern unmittelbar oder mittelbar über die Prüflogik Austauschmittel ansteuerbar sind bzw. angesteuert werden, mit denen als fehlerhaft identifizierte Kunststoffspritzgussteile vor oder nach der Anordnung zu den geprüften Untergruppen aussortiert und durch kavitätereine Kunststoffspritzgussteile ersetzt werden, sodass im Ergebnis die Endverpackungseinheiten nur mit seitenfehlerfreien Kunststoffspritzgussteilen kavitätenrein oder kavitätensortiert bestückt werden.

Das erfindungsgemäße Verfahren (sowie die zur Ausführung des Verfahrens ausgebildete Vorrichtung) hat/haben gegenüber dem Stand der Technik erheblich Vorteile. Zum ersten Mal ist eine 100%-Seitenprüfung, insbesondere auf Form- und/oder Oberflächenfehler auch bei zuvor zu Untergruppen, insbesondere kavitätenreinen Feldern zusammengesetzten, als Pipettenspitzen oder medizinische Reaktionsgefäße ausgebildeten Kunststoffspritzgussteilen, also bei Gewährleistung einer kavitätenreinen oder kavitätensortierten Verpackung in Endverpackungseinheiten möglich. Hierdurch wird die Produktsicherheit erheblich erhöht, da auch sporadische Seitenfehler von in Endverpackungen verpackten Kunststoffspritzgussteilen sicher vermieden werden können. Neben der Gewährleistung einer 100%-Seitenprüfung bei gleichzeitiger gewährleistung einer kavitätenreinen oder kavitätensortieren Verpackung in Endverpackungen ermöglicht die Erfindung eine statistische Zuordnung von mittels der Seitenprüfung ermittelten Fehlern, insbesondere Form- und/oder Oberflächenfehlern zu bestimmten Kavitäten. Es ist also möglich und bevorzugt auszuwerten bzw. nachzuvollziehen, aus welchen Kavitäten bestimmte Fehler bzw. fehlerhafte Kavitäten stammen.

Wesentlich ist, dass es sich bei den in Rede stehenden Verpackungseinheiten bzw. Endverpackungseinheiten, in denen entweder die geprüften Untergruppen gebildet werden oder die nach vorhergehender Bildung der geprüften Untergruppen in einen Zwischenspeicher jeweils mit einer einzigen oder mehreren (nicht sämtlichen) kavitätenreinen, geprüften Untergruppen befüllt werden, um Endverpackungseinheiten handelt, die nach der vollständigen Befüllung zu Endkunden ausgeliefert werden, wobei es sich bei dem Auslieferungsschritt an den Endkunden bzw. die Übergabe an eine Spedition od. dgl. Transporteinheit um einen weiterbildungsgemäßen Verfahrensschritt handelt, der hiermit als beanspruchbar offenbart gelten soll.

In Weiterbildung der Erfindung ist mit Vorteil vorgesehen, dass die Subgruppen mit ihren, bevorzugt linienförmig, hintereinander angeordneten Kunststoffspritzgussteilen mittels der Transfermittel entlang einer Prüfspur durch die Seitenprüfeinrichtung gefördert werden, insbesondere derart, dass eine, bevorzugt horizontale, optische Achse der mindestens einen digitalen Kamera, bevorzugt falls vorgesehen, die optischen Achsen mehrerer in Umfangsrichtung, ganz besonders gleichmäßig, beabstandeter digitaler Kameras, die, bevorzugt vertikale, Längsmittelachse jeweils eines der Kunststoffspritzgussteile in einer auf der Prüfspur liegenden Prüfposition, bevorzugt senkrecht, schneidet/schneiden. Bei einer derartigen Ausführungsform wird jeweils das durch die Prüfposition bewegte Kunststoffspritzgussteil in der Prüfposition von der mindestens einen Digitalkamera erfasst/aufgenommen und auf, sporadische, Seitenfehler geprüft.

Um einen möglichst schnellen Transport der Subgruppen durch die Seitenprüfeinrichtung zu ermöglichen, ist in Weiterbildung der Erfindung vorgesehen, dass die Transfermittel zum Fördern der Subgruppen durch die Seitenprüfeinrichtung einen ersten und einen zweiten Werkstückträger zur jeweiligen Aufnahme einer der S Subgruppen umfasst, wobei die Werkstückträger entlang einer Transferachse zwischen einer jeweiligen Beladeposition, in der sie mit einer der S Subgruppen aus den Untergruppen der ersten Ablageebene beladen werden und einer jeweiligen Entladeposition, aus der die jeweilige Subgruppe nach der optischen Prüfung in die zweite Ablageebene zur (erneuten) kavitätenreinen Untergruppenbildung bzw. untergruppenrekonfiguration überführt wird, hin und her verstellt werden, und dass der erste und der zweite Werkstückträger während des Transfers von der jeweiligen Beladeposition zur jeweiligen Entladeposition mit einer der S Subgruppen entlang einer gemeinsamen Prüfspur durch die Seitenprüfeinrichtung gefördert werden. Anders ausgedrückt sind zwei Werkstückträger vorgesehen, die wechselseitig jeweils eine Subgruppe durch die Seitenprüfeinrichtung fördern, wobei einer der Werkstückträger mit einer zu prüfenden Subgruppe beladen wird, während der andere Werkstückträger nach erfolgter Prüfung der darauf befindlichen Subgruppe entladen wird. Hierdurch kann die Zykluszeit deutlich optimiert werden. Der entladene Werkstückträger fährt dann leer in seine Beladeposition zurück, während der beladende Werkstückträger entlang der Prüfspur durch die Seitenprüfeinrichtung hin zu seiner Entladeposition gefördert wird. Im Hinblick auf die Möglichkeit einer festen Kameraanordnung ist vorgesehen, dass sich beide Werkstückträger innerhalb der Prüfeinrichtung eine Prüfspur teilen, d.h. auf derselben Spur/Achse (d.h. einer gemeinsamen Prüfspur) durch die Prüfeinrichtung gefördert werden, während die Prüfung der darauf befindlichen Kunststoffspritzgussteile mittels der mindestens einen Digitalkamera erfolgt.

Im Hinblick auf die Realisierung der Hin- und Herbewegung der Werkstückträger gibt es unterschiedliche Möglichkeiten. Besonders bevorzugt ist es, wenn die Hin- und Herbewegung des ersten und zweiten Werkstückträgers zwischen ihren jeweiligen Ent- und Beladepositionen/Position in einer gekoppelten Bewegung erfolgt. Dabei kann die Bewegung, beispielsweise durch das Vorsehen eines gemeinsamen, hin und her bewegbaren Riementriebs oder Kettentriebs mechanisch gekoppelt sein. Dies hat den Vorteil, dass nur ein einziger, gemeinsamer, bevorzugt elektrischer, Antrieb vorgesehen werden muss. Alternativ kann für den Fall des Vorsehens mehrerer, beispielsweise servomotorischer Antriebe vorgesehen werden, diese elektronisch zu koppeln, d.h. zu synchronisieren.

Um trotz des Vorsehens einer gemeinsamen Prüfspur für den ersten und den zweiten Werkstückträger auf seinen jeweiligen Prüfweg durch die Seitenprüfeinrichtung eine Kollision der Werkstückträger bei ihrer Begegnung in der Prüfeinrichtung zu vermeiden, ist in Weiterbildung der Erfindung vorgesehen, dass zumindest einer der Werkstückträger, mit Verstellmitteln relativ, insbesondere senkrecht, winklig oder in einer Schwenkbewegung zu der Transferachse verstellt wird, derart, dass die Werkstückträger in der Prüfeinrichtung entlang der Transferachse aneinander vorbei verstellt werden, während sich der mit einer der S Subgruppen beladene Werkstückträger der Werkstückträger in der Prüfspur befindet. Bevorzugt ist es, wenn die Werkstückträger in einer gemeinsamen Horizontalebene aneinander vorbeibewegt werden, wobei grundsätzlich auch ein übereinanderaneinandervorbeibewegen, also ein Aneinandervorbeifahren mit vertikalem Versatz realisierbar ist, durch das Vorsehen entsprechender Hubverstellmittel. Bevorzugt ist eine Ausführungsform, bei welcher jedem der Werkstückträger Verstellmittel zugeordnet sind, sodass jeder der Werkstückträger in die Prüfspur hinein und wieder aus dieser heraus verstellbar ist, bevorzugt in bzw. aus einer eigenen Spur. In diesem Fall sind dann vorzugsweise drei parallele Spuren vorgesehen.

Wie erwähnt, begegnen sich die Werkstückträger bei ihrer Hin- und Herbewegung innerhalb der Prüfeinrichtung, bevorzugt während der Prüfung der Kunststoffspritzgussteile mittels der mindestens einen Kamera. Um trotz, bevorzugt in einer Horizontalebene, aneinander vorbeizubewegender Werkstückträger eine Prüfung der Kunststoffspritzgussteile im befüllten Werkstückträger zu ermöglichen bzw. zu vereinfachen ist in Weiterbildung der Erfindung vorgesehen, dass die Werkstückträger jeweils eine, insbesondere beidseitige, Seitenöffnung aufweisen, in die die Kunststoffspritzgussteile einer in dem jeweiligen Werkstückträger angeordneten Subgruppe, bevorzugt von oben vertikal, hineinragen, insbesondere diese vertikal durchsetzen und von der mindestens einen Kamera durch die Seitenöffnung hindurch optisch geprüft werden. Im Hinblick auf die konkrete Ausbildung der Seitenöffnung(en) gibt es viele Möglichkeiten. Bevorzugt ist jeder Werkstückträger zu beiden Seiten senkrecht zur Transferachse offen bzw. weist dort eine Seitenöffnung auf. Ganz besonders bevorzugt ist jeder Werkstückträger mit einem senkrecht zur Transferachse offenen Durchbruch versehen, um somit mit mindestens einer digitalen Kamera, insbesondere mit mindestens zwei zu beiden Seiten der Prüfspur angeordneten digitalen Kameras jeweils die Mantelfläche der Kunststoffspritzgussteile erfassen zu können. Die seitlichen Öffnungen in den Werkstückträgern fluchten bevorzugt senkrecht zur Transferachse beim aneinander Vorbeibewegen der Werkstückträger in der Prüfeinrichtung.

Ganz besonders bevorzugt ist es dabei die mindestens eine digitale Kamera, ganz besonders bevorzugt mehrere digitale Kameras der Seitenprüfeinrichtung derart anzuordnen, dass die Kunststoffspritzgussteile im ersten Werkstückträger durch die Seitenöffnung des zweiten Werkstückträgers hindurch optisch geprüft werden und/oder die Kunststoffspritzgussteile im zweiten Werkstückträger durch die Seitenöffnung des ersten Werkstückträgers hindurch.

Wie erläutert, betrifft die Erfindung auch eine Verpackungsvorrichtung zur Ausführung eines erfindungsgemäßen Verfahrens. Hierzu weist die Vorrichtung entsprechende Funktionsmittel auf, die derart ausgebildet und von Steuermitteln angesteuert sind, dass diese die jeweiligen Verfahrensschritte ausführen. So umfasst die erfindungsgemäße Verpackungsvorrichtung mindestens einen, Entnahmegreifer, der ausgebildet und von Steuermitteln angesteuert ist zum mehrfachen, jeweils gleichzeitigen Entnehmen eines jeweiligen Schusses von K Kunststoffspritzgussteilen aus K Kavitäten einer (nicht zur Verpackungsvorrichtung gehörenden, jedoch bevorzugt im Rahmen des Systems daneben angeordneten bzw. anordnenbaren) Spitzgussvorrichtung, deren Werkzeuge zur Herstellung von Pipettenspitzen oder medizinischen Reaktionsgefäßen ausgebildet sind und bei der vorzugsweise die K Kavitäten, bevorzugt gleichmäßig, auf C Cluster verteilt angeordnet sind. Ferner umfasst die Verpackungsvorrichtung Untergruppenbildungsmittel zur Bildung von kavitätenreinen Untergruppen in der ersten Ablageebene. Die Vorrichtung umfasst zudem Mittel zum kavitätenreinen und/oder kavitätensortierten Auffüllen von Endverpackungseinheiten. Für den Fall der Ausbildung der zweiten Ablageebene als Zwischenspeicher können diese Mittel als Übergabegreifer ausgebildet sein oder einen solchen umfassen, um die geprüften Untergruppen von der zweiten Ablageebene in die Endverpackungseinheiten zu transferieren bzw. zu übergeben. Für den Fall der Realisierung einer Direktbeladung umfassen die Mittel bevorzugt einen zweiten Subgruppengreifer oder werden von diesem gebildet, mit dem die Subgruppen direkt in die Endverpackungseinheiten abgelegt werden können, also die kavitätenreinen, geprüften Untergruppen unmittelbar in den Endverpackungseinheiten gebildet werden.

Die Vorrichtung zeichnet sich aus durch erste und zweite Subgruppengreifer zum Be- bzw. Entladen der Transfermittel, mit denen Subgruppen durch die Prüfeinrichtung der Verpackungsvorrichtung förderbar sind.

Sämtliche im Rahmen der Offenbarung genannten Greifer bzw. Greifermittel können beispielsweise als mechanische Greifer oder bevorzugt als Vakuum- bzw. Unterdruckgreifer ausgebildet sein, wobei letztere die Kunststoffspritzgussteile durch Ansaugen während der Überführung zwischen zwei Positionen fixieren.

Die Verpackungsvorrichtung zeichnet sich insbesondere durch die vorerwähnte optische Seitenprüfeinrichtung, umfassend mindestens eine Digitalkamera aus, entlang derer, insbesondere sämtliche, zuvor zu kavitätenreinen Untergruppen in der ersten Ablageebene sortierte bzw. zusammengefasste Kunststoffspritzgussteile in Subgruppen mit, insbesondere linienförmig, hintereinander angeordneten (nicht zur Vorrichtung gehörenden) Kunststoffspritzgussteilen zu Prüfzwecken gefördert werden.

Im Hinblick auf die konkrete Ausgestaltung der Verstellmittel zur Verstellung mindestens eines bevorzugt vorgesehenen Werkstückträgers senkrecht zur Transferachse zur Kollisionsvermeidung während des aneinander Vorbeibewegens der Werkstückträger in der Prüfeinrichtung gibt es unterschiedliche Möglichkeiten. Bevorzugt ist es, wenn die Verstellmittel einen senkrecht zur Transferachse verstellbaren Schlitten umfassen, auf dem der jeweilige Werkstückträger angeordnet ist und über den der Werkstückträger dann in die Prüfspur hinein- und wieder aus dieser herausverstellbar ist. Der Schlitten kann beispielsweise pneumatisch oder servomotorisch angetrieben werden, wobei für den bevorzugten Fall des Vorsehens eines Riementriebs ein Schlittenträger am Riemen fixiert ist.

Grundsätzlich ist es möglich nur einen der Werkstückträger auf einem, beispielsweise pneumatisch oder servomotorisch antreibbaren Schlitten anzuordnen - bevorzugt ist eine Ausführungsform, bei der jeder Werkstückträger auf einem entsprechend verstellbaren Schlitten angeordnet ist, wobei in diesem Fall bevorzugt beide Schlitten über jeweils einen Schlittenträger am Riementrieb fixiert sind.

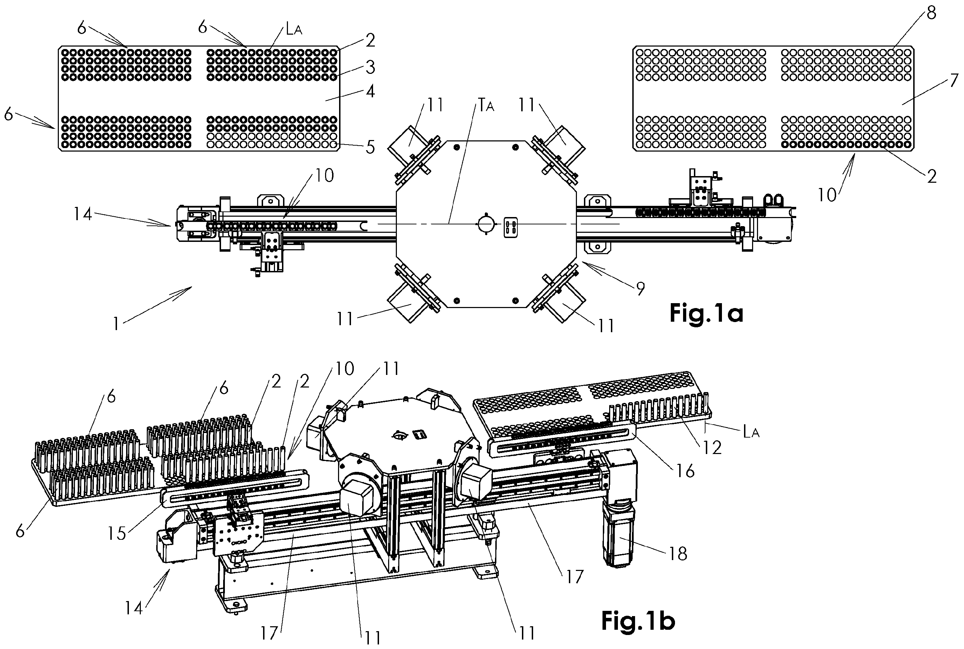

Darüber hinaus betrifft die Erfindung ein Spritzgusssystem, umfassend eine wie zuvor beschrieben ausgebildete Spritzgussvorrichtung zum Herstellen von Pipettenspitzen oder medizinischen Reaktionsgefäßen, bei der vorzugsweise die K Kavitäten, bevorzugt gleichmäßig, auf C Cluster verteilt angeordnet sind sowie eine erfindungsgemäße Verpackungsvorrichtung. Im Hinblick auf eine bevorzugte Ausbildung und Anordnung der C Cluster wird ausdrücklich auf die veröffentlichte WO 2022/022955 A1 der Anmelderin verwiesen. Dort ist beispielhaft eine typische Kavitätenanordnung in Clustern in Fig. 1 gezeigt, wobei selbstverständlich die Anzahl der Cluster sowie die Anzahl der Kavitäten pro Cluster variieren kann.

Weitere Vorteile, Merkmale und Einzelheiten der Erfindung ergeben sich aus der nachfolgenden Beschreibung eines bevorzugten Ausführungsbeispiels sowie anhand der Zeichnungen.

Diese zeigen in:
- Fig. 1a:: ausschnittsweise eine Verpackungsvorrichtung mit optischer Seitenprüfeinrichtung in einer Draufsicht, wobei in Fig. 1a sowohl ausschnittsweise eine erste Ablageebene mit kavitätenreinen Untergruppen, gezeigt ist als auch eine zweite Ablageebene, in der gerade Subgruppe für Subgruppe geprüfte, kavitätenreine Untergruppen gebildet werden, wobei die zweite Ablageebene in dem gezeigten Ausführungsbeispiel von einem statischen oder verstellbaren Zwischenspeicher gebildet wird, aus welchem dann nicht gezeigte Endverpackungseinheiten mit jeweils einer oder mehrerer geprüften, kavitätenreiner Untergruppen beladen werden, wobei alternativ eine Direktbeladung möglich ist, wobei in diesem Fall die zweite Ablageebene unmittelbar von den Endverpackungseinheiten gebildet wird,
- Fig. 1 b:: eine perspektivische Seitenansicht der Darstellung gemäß Fig. 1a,
- Fig. 2a - Fig. 2c:: unterschiedliche Ansichten der optischen Seitenprüfeinrichtung sowie der Transfermittel während der Beladung eines ersten Werkstückträgers,
- Fig. 3a - Fig. 3c:: unterschiedliche Darstellungen der Einheiten gemäß Fig. 2a bis Fig. 2c mit beladenem Werkstückträger zu Beginn des Transfers,
- Fig. 4a - Fig. 4d:: teilweise vergrößerte Ansichten der Einheiten gemäß Fig. 3a bis Fig. 3c während der Prüfung der Kunststoffspritzgussteile beim Durchfahren der Seitenprüfeinrichtung mittels des mit einer Subgruppe befüllten Werkstückträgers auf einer gemeinsamen Prüfspur für die Werkstückträger,
- Fig. 5a - Fig. 5c:: unterschiedliche Darstellungen der Einheiten gemäß den vorhergehenden Figuren beim Herausbewegen des (noch) mit einer geprüften Subgruppe befüllten Werkstückträgers aus der gemeinsamen Prüfspur und des Hineinbewegens des im nächsten Schritt zu befüllenden Werkstückträgers in die gemeinsame Prüfspur, und
- Fig. 6a:: die Einheiten aus den vorhergehenden Figuren bei wechselseitigem Be- und Entladen der Werkstückträger.

In den Figuren sind gleiche Elemente und Elemente mit der gleichen Funktion mit den gleichen Bezugszeichen gekennzeichnet.

In den Figuren, insbesondere den Fig. 1a und 1b ist ausschnittsweise eine Verpackungsvorrichtung 1 für als Pipettenspitzen oder medizinische Reaktionsgefäße ausgebildete, bevorzugt rotationssymmetrische Kunststoffspritzgussteile 2 gezeigt. Diese weisen eine jeweils bevorzugt vertikale und in dem gezeigten Ausführungsbeispiel in Fig. 1 senkrecht zur Zeichnungsebene orientierte Längsmittelachse L_{A} auf. Die jeweilige Längsmittelachse L_{A} verläuft zentrisch durch eine jeweilige, vorliegend obere, als Einfüllöffnung ausgebildete Öffnung 3.

In Fig. 1a ist ausschnittsweise eine erste Ablageebene 4 in Form eines horizontalen Zwischenspeichers gezeigt, umfassend Aufnahmen 5 für die Kunststoffspritzgussteile 2. Nicht gezeigt ist eine nicht zur Verpackungsvorrichtung, jedoch zum Spritzgusssystem gehörende Spritzgussvorrichtung, die der Verpackungsvorrichtung bevorzugt vorgeordnet ist. Ebenso nicht gezeigt sind mindestens ein Entnahmegreifer sowie Steuermittel zum Ansteuern des Entnahmegreifers (und andere Funktionseinheiten) zum mehrfachen, jeweils gleichzeitigen Entnehmen eines jeweiligen Schusses von K Kunststoffspritzgussteilen aus K Kavitäten der Spritzgussvorrichtung, bei der bevorzugt die K Kavitäten, bevorzugt gleichmäßig, auf C Cluster verteilt angeordnet sind. Ebenso nicht gezeigt sind Untergruppenbildungsmittel, die ausgebildet und von Steuermitteln angesteuert sind zum gleichmäßigen und kavitätenreinen Verteilen der K Kunststoffspritzgussteile (einzeln oder in Unter-Untergruppen) auf U kavitätenreine Untergruppen 6 in der ersten Ablageebene 4. Auch nicht gezeigt sind Mittel zum kavitätenreinen oder kavitätensortierten Auffüllen der Endverpackungseinheiten.

Im Hinblick auf konkrete Möglichkeiten zur Ausbildung der nicht dargestellten Elemente bzw. Einheiten und Funktionsmittel wird auf die, insbesondere in der Beschreibungseinleitung erwähnten, früheren Anmeldungen und Patente der Anmelderin verwiesen.

In Fig. 1a ist rechts von der ersten Ablageebene 4 eine zweite Ablageebene 7 in Form eines horizontalen Zwischenspeichers gezeigt, wobei die zweite Ablageebene 7 alternativ unmittelbar von nicht gezeigten Endverpackungen gebildet sein kann. Die zweite Ablageebene 7 weist ebenfalls Aufnahmen 8 zur Aufnahme von in diesem Fall mittels einer mit einer optischen Seitenprüfeinrichtung 9 auf sporadische Fehler geprüfte Kunststoffspritzgussteilen 2 auf. In der zweiten Ablageebene 7 werden die geprüften Kunststoffspritzgussteile 2, genauer Subgruppen 10 von linienförmig hintereinander angeordneten Kunststoffspritzgussteilen 2 erneut zu kavitätenreinen Untergruppen, vorliegend geprüften k Untergruppen zusammengesetzt, wobei zumindest im Falle einer erfolgreichen Prüfung, d.h. bei Nichterkennung von Fehlern die Zusammensetzung der geprüften Untergruppen in der zweiten Ablageebene 7 der Zusammensetzung der noch nicht geprüften kavitätenreinen Untergruppen in der ersten Ablageebene 4 entspricht. Grundsätzlich ist es dabei denkbar, dass die Reihen in den geprüften Untergruppen identisch angeordnet werden wie in den entsprechenden Untergruppen in der ersten Ablageebene 4, wobei alternativ auch eine andere Reihenfolge der Reihen, insbesondere Subgruppen der jeweiligen Untergruppe erfolgen kann, insbesondere eine Anordnung in umgekehrter Reihenfolge. Die Zusammensetzung bleibt (grundsätzlich, d.h. unabhängig vom gezeigten Ausführungsbeispiel) gleich, d.h. die zu einer der Untergruppen in der ersten Ablageebene gehörenden Subgruppen werden wieder zu einer gemeinsamen Untergruppe zusammengesetzt, wobei einzelne Kunststoffspritzgussteile nach Fehlererkennung ausgetauscht werden können.

In Fig. 1a ist zu erkennen, dass die Untergruppen 6 in der ersten Ablageebene 4 als auch dann später die geprüften Untergruppen in der zweiten Ablageebene 7 jeweils geometrisch als Felder ausgebildet sind, jeweils aufweisend eine rechteckige Hüllkontur. Jede Untergruppe 6 in der ersten Ablageebene 4 als auch dann später jede geprüfte Untergruppe 6 in der zweiten Ablageebene 7 besteht aus mehreren, vorliegend von links nach rechts sowie parallel zu einer später noch zu erläuternden Transferachse T_{A} angeordneten Reihen von linienförmig hintereinander angeordneten Kunststoffspritzgussteilen 2, wobei jede Reihe eine zu prüfende Subgruppe 10 bildet, die entlang der Transferachse T_{A} durch die Seitenprüfeinrichtung 9 transferiert wird.

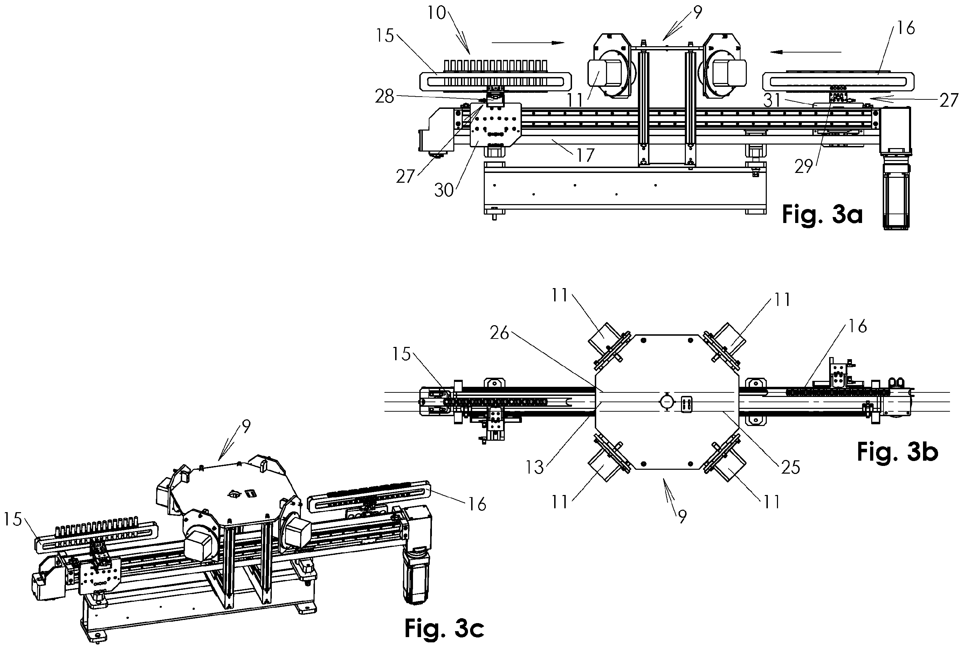

In dem gezeigten Ausführungsbeispiel umfasst die Seitenprüfeinrichtung 9 vier gleichmäßig (hier im 90° Winkel) in Umfangsrichtung beanstandete digitale Kameras 11 zum Erfassen der sich um die jeweilige Längsmittelachse L_{A} eines Kunststoffspritzgussteils 2 erstreckende Seiten- bzw. Mantelfläche 12 der Kunststoffspritzgussteile 2. Die digitalen Kameras 11 sind auf zwei Seiten einer gemeinsamen Prüfspur 13 (vgl. beispielsweise Fig. 3b) verteilt angeordnet.

Die Verpackungsvorrichtung 1 umfasst Transfermittel 14 zum Transferieren von kavitätenreinen Subgruppen 10 durch die Seitenprüfeinrichtung 9.

Die Transfermittel 14 umfassen in dem gezeigten Ausführungsbeispiel einen ersten 15 und einen zweiten Werkstückträger 16 zur jeweiligen Aufnahme einer Subgruppe 10. Die beiden Werkstückträger 15, 16 sind in einer mechanisch gekoppelten Bewegung, vorliegend mittels eines Riementriebs 17, umfassend einen gemeinsamen elektromotorischen Antrieb hin- und her bewegbar, zwischen einer jeweiligen Beladeposition (in den Fig. 1a und Fig. 1b links sowie einer jeweiligen Entladeposition, in den Fig. 1a und Fig. 1b rechts). In der Beladeposition wird ein jeweils leerer, vorliegend der erste Werkstückträger 15 mit einer Subgruppe 10 von linienförmig hintereinander angeordneten Kunststoffspritzgussteilen 2 beladen, wobei vorliegend jede Subgruppe 10 einer Reihe von Kunststoffspritzgussteilen 2 einer zugehörigen Untergruppe 6 entspricht. Die Aufteilung der Untergruppen 6 in Subgruppen 10 und die Beladung der Werkstückträger 15, 16 mit den Subgruppen 10 erfolgt mittels eines nicht gezeigten ersten Subgruppengreifers, während die Entladung des sich jeweils in einer Entladeposition befindlichen Werkstückträgers 15, 16 mit einem ebenfalls nicht gezeigten zweiten Subgruppengreifer erfolgt, mit welchem die Subgruppen 10 in der zweiten Ablageebene 7 wieder zu dann geprüften, kavitätenreinen Untergruppen zusammengesetzt werden.

Nicht gezeigt sind bevorzugt vorgesehene von der Prüflogik der Seitenprüfeinrichtung angesteuert bzw. mit dieser zusammenwirkende Austauschmittel, umfassend Aussortiermittel zum Aussortieren von als fehlerhaft erkannten Kunststoffspritzgussteilen 2 und zum Ersetzen des freigewordenen Platzes durch kavitätenreine, d.h. zur jeweiligen Untergruppe gehörende bzw. aus der jeweils selben Kavität stammende, fehlerfreie, bevorzugt bereits geprüfte Kunststoffteile, die aus einem entsprechenden Vorrat entnommen werden können.

In den Figuren ist zu erkennen, dass die Werkstückträger 15, 16 seitlich, d.h. senkrecht zur Transferachse T_{A} offen sind, sprich seitliche Öffnungen 19, 20 aufweisen, in die die zu prüfenden Kunststoffspritzgussteile 2 einer jeweiligen Subgruppe 10 hineinragen, vorliegend diese durchragen, um dann seitlich durch die seitlichen Öffnungen 19, 20 mit den Kameras 11 erfasst zu werden. Die seitlichen Öffnungen 19, 20 sind vorliegend gemäß einer bevorzugten Ausführungsform durch als Durchbrüche, d.h. Durchgangsöffnungen in einem jeweiligen Werkstückträgerrahmen 21, 22 ausgebildet.

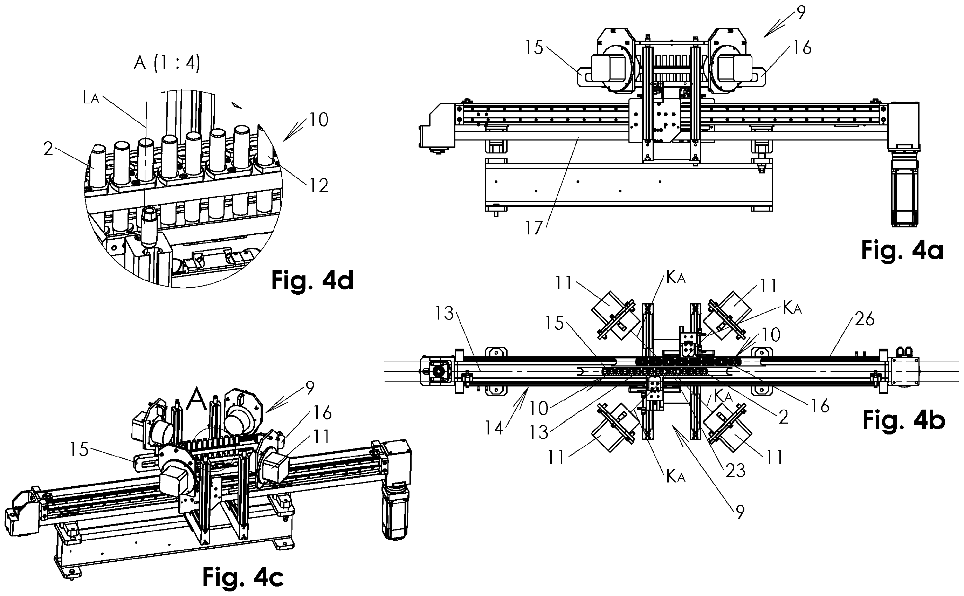

Wie in den Fig. 4a bis Fig. 4d gezeigt, werden die zu prüfenden Kunststoffspritzgussteile 2 einer zu prüfenden Subgruppe 10 durch die seitlichen Öffnungen 19, 20 von den Kameras 11 mit Blick auf die jeweilige Mantelfläche geprüft, und zwar in einer in Fig. 4b angezogenen Prüfposition 23, in welcher sich Kameraachsen K_{A} der Digitalkameras 11 treffen und die Längsmittelachse L_{A} des sich jeweils in der Prüfposition 23 befindlichen Kunststoffspritzgussteils 2 einer Subgruppe 10 winklig schneiden, vorliegend senkrecht.

Die Prüfposition 23 befindet sich auf einer gemeinsamen Prüfspur 13 für beide Werkstückträger 15, 16, die die Werkstückträger 15, 16 zum Prüfen der jeweils aufgenommenen Subgruppe 10 durchlaufen.

Aus Fig. 4a bis Fig. 4d wird insbesondere auch deutlich, dass die Werkstückträger 15, 16 in der Prüfeinrichtung 9 aneinander vorbeibewegt werden. In dem gezeigten Ausführungsbeispiel werden die auf dem ersten Werkstückträger 15 befindlichen Kunststoffspritzgussteile 2 durch dessen seitliche Öffnung 19 hindurch geprüft sowie durch die mit der seitlichen Öffnung 19 fluchtende seitliche Öffnung 20 im vorbeibewegten zweiten Werkstückträger 16. Die seitlichen Öffnungen 19, 20 in den Werkstückträgern 15, 16 fluchten senkrecht zur Transferachse T_{A} beim aneinander Vorbeibewegen der Werkstückträger in der Prüfeinrichtung 9.

In dem konkreten Ausführungsbeispiel umfassen die Transfermittel 14 zusätzlich zu der gemeinsamen Prüfspur 13 eine erste Rücklaufspur 25 für den ersten Werkstückträger 15 sowie eine zweite Rücklaufspur 26 für den zweiten Werkstückträger 16. Die Werkstückträger 15, 16 sind mithilfe von Verstellmitteln 27 zwischen der gemeinsamen Prüfspur 13 und der jeweiligen Rücklaufspur 25, 26 senkrecht zur Transferachse verstellbar.

In dem konkreten Ausführungsbeispiel umfassen die Verstellmittel 27 für jeden Werkstückträger 15, 16 einen ersten bzw. zweiten Schlitten 28 bzw. 29, der vorliegend jeweils pneumatisch aktuierbar ist. Jeder Schlitten 28, 29 ist mit einem zugehörigen Träger 30, 31 mit einer jeweiligen Seite des Riementriebs 17 fest verbunden.

Denkbar ist es alternativ auch nur einen der Werkstückträger 15, 16 zwischen der gemeinsamen Prüfspur 13 und einer davon beabstandeten, parallelen Spur, beispielsweise mittels eines Schlittens 28, 29 verstellbar anzuordnen.

Wesentlich ist, dass die Verstellung eines einzigen Werkstückträgers 15, 16 oder beider Werkstückträger 15, 16 derart erfolgt, dass diese trotz, dass sich einer der Werkstückträger 15, 16 in der Prüfeinrichtung auf der gemeinsamen Prüfspur 13 befindet, kollisionsfrei aneinander vorbeibewegt werden können.

Auch ist variierbar, ob die jeweilige Beladeposition beispielsweise auf der gemeinsamen Prüfspur 13 bzw. Prüfachse angeordnet ist oder auf einer dazu parallelen Spur, insbesondere Rücklaufspur 25, 26. Ebenso verhält es sich analog mit der Entladeposition für jeden Werkstückträger 15, 16, die alternativ auf der gemeinsamen Prüfspur 13 bzw. -achse angeordnet sein kann oder in einer hierzu parallelen Spur, insbesondere Rücklaufspur 25, 26. Je nach Beladeposition muss der zugehörige Greifer, also der erste bzw. zweite Subgruppengreifer lediglich entsprechend angesteuert werden bzw. sein.

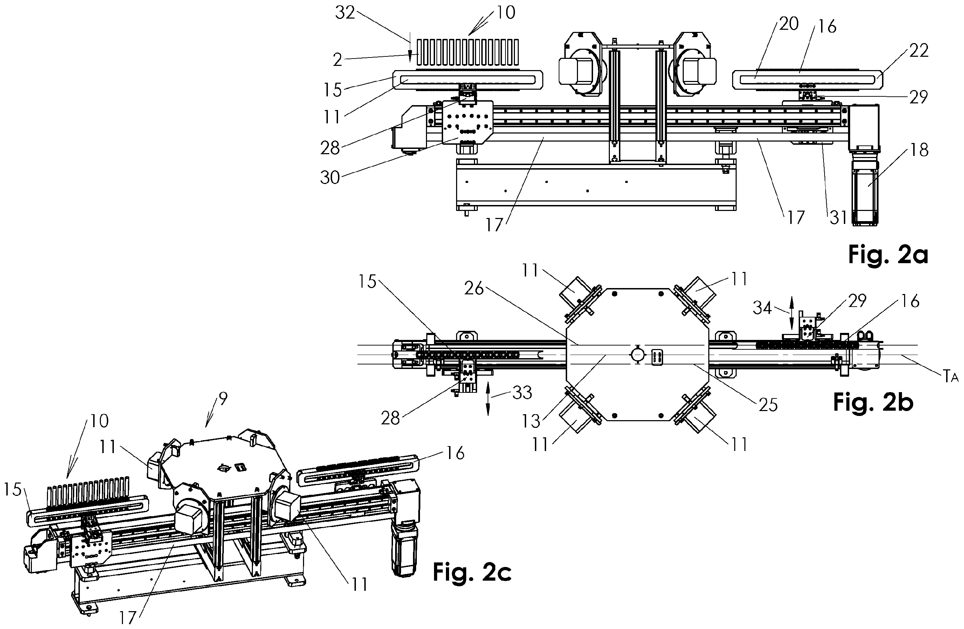

In den Fig. 2a bis 2c ist der Verfahrensschritt des Beladens, vorliegend des ersten, Werkstückträgers 15 mit einer kavitätenreinen Subgruppe 10 von entlang der Transferachse T_{A} hintereinander angeordneten Kunststoffspritzgussteilen 2 mittels des nicht dargestellten ersten Subgruppengreifers gezeigt, wobei die Kunststoffspritzgussteile 2 entlang des Pfeils 32, also in vertikaler Richtung von oben nach unten in den Werkstückträger 15 eingesetzt werden und danach dessen seitliche Öffnung 19 durchsetzen. Aus Fig. 2b ist die zugehörige Beladeposition des ersten Werkstückträgers 15 zu erkennen. Diese befindet sich vorliegend beispielhaft in der Zeichnungsebene am linken Ende der gemeinsamen Prüfspur 13. Aus Fig. 2b ist die Verstellmöglichkeit des ersten Werkstückträgers 15 mit den Verstellmitteln 27, vorliegend dem ersten Schlitten 28 gezeigt, und zwar senkrecht zur Transferachse T_{A} in den Pfeilrichtungen 33, und zwar konkret zwischen der gemeinsamen Prüfspur 13 und der zugehörigen Rücklaufspur 25. Analog ist der zweite Werkstückträger 16 mittels der Verstellmittel 27, konkret dem zweiten **S**chlitten 29 ebenfalls senkrecht zur Transferachse T_{A} verstellbar, und zwar zwischen der gemeinsamen Prüfspur 13 und der dem zweiten Werkstückträger 16 zugeordneten zweiten Rücklaufspur 26. Hierzu ist der Werkstückträger 16 mittels des Verstellschlittens 19 in den Pfeilrichtungen 34 verstellbar.

Zu erkennen ist, dass sich die Entladeposition des zweiten Werkstückträgers 16 beispielhaft auf dessen Rücklaufspur 26 befindet.

Die Fig. 3a bis Fig. 3c entsprechen im Wesentlichen den Fig. 2a bis 2c, mit dem Unterschied, dass die Beladung des ersten Werkstückträgers 15 mit einer Subgruppe 10 bereits erfolgt ist und, wie durch die Pfeile in Fig. 3a angedeutet ist, die Werkstückträger 15, 16 in aneinander entgegengesetzte Richtungen entlang der Transferachse T_{A} verstellt werden, vorliegend in Richtung der Seitenprüfeinrichtung 9 mittels des Riementriebs 17. Im Gegensatz zu der Situation in den Fig. 3a bis 3c befinden sich die Werkstückträger 15, 16 bei der Situation in den Fig. 4a bis Fig. 4d in der Seitenprüfeinrichtung 9 und die Prüfung auf Form- und/oder Oberflächenfehler durch die mantelseitige Aufnahme der Kunststoffspritzgussteile 2 mittels der Digitalkameras 11 ist im Gange. Der erste Werkstückträger 15 befindet sich auf der gemeinsamen Prüfspur 13 während der leere, zweite Werkstückträger 16 den ersten Werkstückträger 15 auf seiner Rücklaufspur 26 passiert. Nacheinander durchlaufen so die Kunststoffspritzgussteile 2 der Subgruppe 10 auf dem ersten Werkstückträger 15 die Prüfposition 23 und werden durch die Analyse der Mantelfläche geprüft.

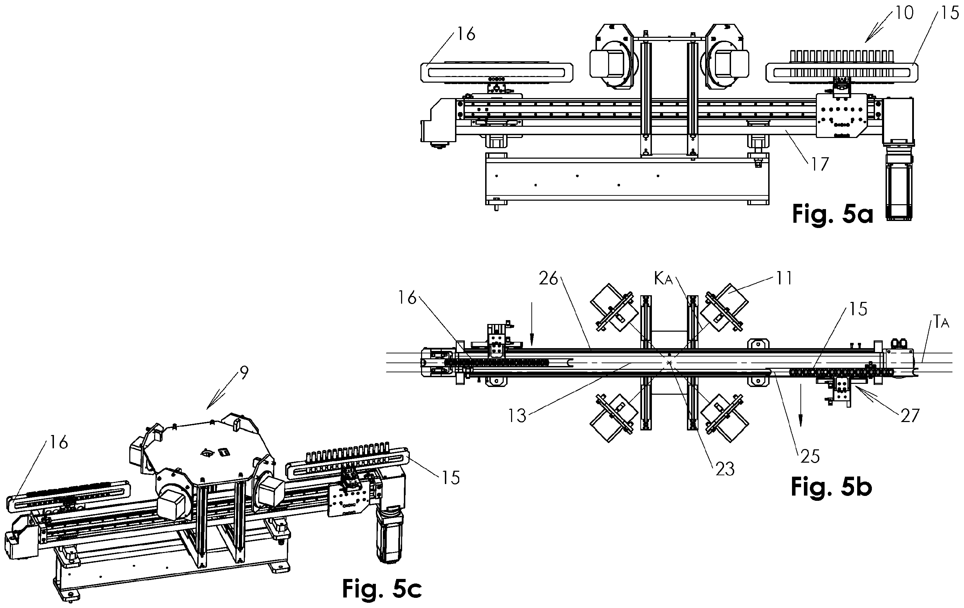

In der Situation gemäß den Fig. 5a bis 5c ist die Prüfung der auf dem ersten Werkstückträger 15 befindlichen Subgruppe 10 erfolgt. Die Werkstückträger 16 befinden sich in einer Endlage, und zwar der zweite Werkstückträger 16 in seiner Beladeposition und der erste Werkstückträger 15 in seiner Entladeposition. In den Endlagen hat ein Spurwechsel stattgefunden. Zu erkennen ist, dass sich der zweite Werkstückträger 16 nun auf der Prüfspur 13 befindet, auf der beispielhaft auch seine Beladeposition realisiert ist. Der erste Werkstückträger 15 wurde mithilfe seines (Verstell-)schlittens 27 auf seine Rücklaufspur 25 bewegt. Angedeutet ist dies durch in der Zeichnung dargestellte Verstellpfeile. Bevorzugt ist eine Verstellung der Werkstückträger 15, 16 senkrecht zu Transferachse T_{A} in den jeweiligen Endlagen der Werkstückträger 15, 16 alternativ bei Vorsehen von Verstellmitteln 27 für nur einen Werkstückträger 15, 16 in den Endlagen dieses Werkstückträgers 15, 16, wobei alternativ auch eine Verstellung während der Verstellbewegung bzw. des Transfers der Werkstückträger 15, 16 entlang der Transferachse T_{A} realisierbar ist.

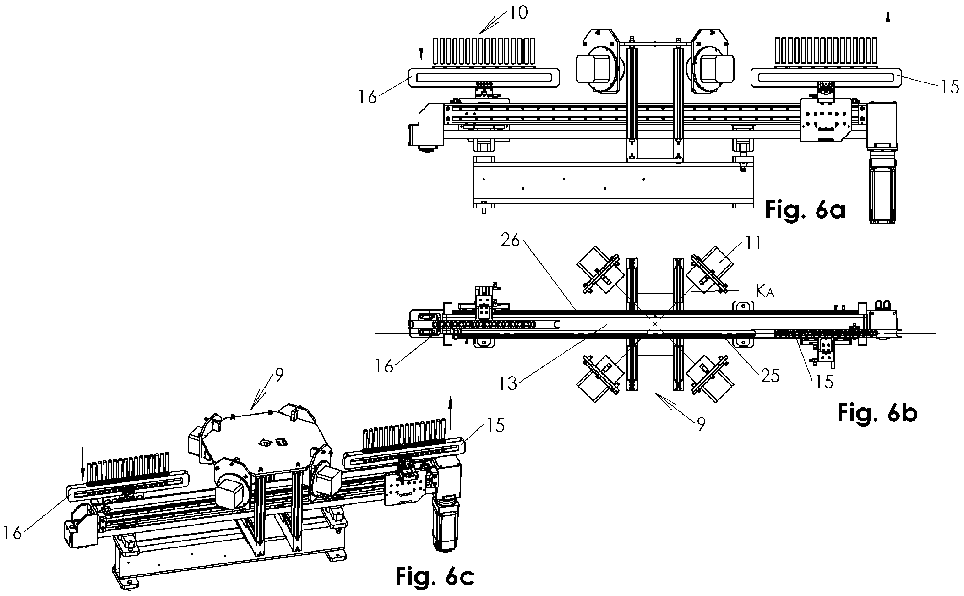

In der Situation gemäß den Fig. 6a bis 6c wird der in seiner Beladeposition befindliche zweite Werkstückträger 16 mit einer noch zu prüfenden Subgruppe 10 von Kunststoffspritzgussteilen 2 beladen, und zwar mittels eines nicht gezeigten ersten Subgruppengreifers, während der erste, sich in seiner Entladeposition befindliche Werkstückträger 15 mittels eines ebenfalls nicht gezeigten zweiten Subgruppengreifers entladen wird und dann mittels des zweiten Subgruppengreifers die Subgruppe 10 auf bzw. in der zweiten Ablageebene 7 abgelegt wird, derart, dass geprüfte, kavitätenreine Untergruppen gebildet werden.

Danach wird dann der zweite Werkstückträger 16 mit seiner zu prüfenden Subgruppe 10 auf der gemeinsamen Prüfspur 13 durch die Seitenprüfeinrichtung 9 bewegt und der erste, dann geleerte Werkstückträger 15 an diesem vorbei in Richtung hin zu seiner Beladeposition, die deckungsgleich sein kann von der Beladeposition des zweiten Werkstückträgers 16 oder parallel versetzt hierzu.

### Bezugszeichen

- 1: Verpackungsvorrichtung
- 2: Kunststoffspritzgussteile
- 3: Öffnung
- 4: 1. Ablageebene
- 5: Aufnahmen
- 6: kavitätenreine Untergruppen
- 7: 2. Ablageebene
- 8: Aufnahmen
- 9: optische Seitenprüfeinrichtung
- 10: Subgruppen
- 11: digitale Kameras
- 12: Mantelfläche
- 13: gemeinsame Prüfspur
- 14: Transfermittel
- 15: 1. Werkstückträger
- 16: 2. Werkstückträger
- 17: Riementrieb
- 18: elektromotorischer Antrieb
- 19: seitliche Öffnungen
- 20: seitliche Öffnungen
- 21: Werkstückträgerrahmen
- 22: Werkstückträgerrahmen
- 23: Prüfposition
- 25: erste Rücklaufspur
- 26: zweite Rücklaufspur
- 27: Verstellmittel für die Werkstückträger
- 28: erster (Verstell-)schlitten
- 29: zweiter (Verstell-)schlitten
- 30: Träger
- 31: Träger
- 32: Pfeilrichtung
- 33: Pfeilrichtungen
- 34: Pfeilrichtungen

- L_{A}: Längsmittelachse
- T_{A}: Transferachse

- K_{A}: Kameraachsen

## Patentansprüche

1. Verpackungsverfahren zum Verpacken von als Pipettenspitzen oder medizinische Reaktionsgefäße ausgebildeten, jeweils eine, bevorzugt eine jeweilige Öffnung (3) zentrisch durchsetzende, Längsmittelachse (L_{A}) aufweisenden, Kunststoffspritzgussteilen (2) in Endverpackungseinheiten, umfassend die Schritte:
- mehrfaches, jeweils gleichzeitiges Entnehmen eines jeweiligen Schusses von K Kunststoffspritzgussteilen (2) aus K Kavitäten, wobei bevorzugt die K Kavitäten, insbesondere gleichmäßig, auf C Cluster verteilt angeordnet sind,
- gleichmäßiges und kavitätenreines Verteilen der K Kunststoffspritzgussteile (2) auf U kavitätenreine Untergruppen (6) in einer ersten Ablageebene (4), bevorzugt bis jede Untergruppe L Kunststoffspritzgussteile (2) aufweist, wobei die Anzahl L der Kunststoffspritzgussteile (2) einer Untergruppe (6) der Anzahl A der in einer Endverpackungseinheit maximal aufnehmbaren Kunststoffspritzgussteilen (2) oder einem ganzzahligen Teiler von A entspricht,
- kavitätenreines oder kavitätensortiertes Auffüllen der Endverpackungseinheiten, bis die Endverpackungseinheiten jeweils A Kunststoffspritzgussteile (2) aufweisen,
**dadurch gekennzeichnet,**
**dass** die Kunststoffspritzgussteile (2) der Untergruppen (6) vor dem Auffüllen der Endverpackungen in einer, mindestens eine, bevorzugt mehrere in einer Umfangsrichtung beabstandete, digitale Kamera/s (11) umfassenden Seitenprüfeinrichtung (9) einer optischen Seitenprüfung, insbesondere auf Form- und/oder Oberflächenfehler, unterzogen werden, und
**dass** hierzu die Untergruppen (6) in jeweils S Subgruppen (10) mit, insbesondere linienförmig, hintereinander angeordneten Kunststoffspritzgussteilen (2) aufgeteilt und die S Subgruppen (10) nacheinander mit Transfermitteln (14) durch die Seitenprüfeinrichtung (9) gefördert und dort der Seitenprüfung unterzogen werden, und
**dass** die S Subgruppen (10) nach Durchlaufen der Seitenprüfeinrichtung (9) in einer zweiten Ablageebene (7) zu geprüften kavitätenreinen Untergruppen angeordnet werden, wobei deren Zusammensetzung für den Fall der Nicht-Aussortierung einzelner Kunststoffspritzgussteile aufgrund einer Fehlererkennung bevorzugt der Zusammensetzung der Untergruppen (6) der ersten Ablageebene (4) entspricht, und wobei die zweite Ablageebene (7) entweder unmittelbar von den Endverpackungseinheiten oder von einem statischen oder verstellbaren, bevorzugt horizontalen, Zwischenspeicher gebildet wird und die geprüften Untergruppen aus dem Zwischenspeicher in die Endverpackungseinheiten überführt werden.

2. Verpackungsverfahren nach Anspruch 1,
**dadurch gekennzeichnet,**
**dass** die Subgruppen (10) mittels der Transfermittel (14) entlang einer Prüfspur (13) durch die Seitenprüfeinrichtung (9) gefördert werden, insbesondere derart, dass eine optische Achse der mindestens einen Kamera (11), bevorzugt die optischen Achsen mehrerer in Umfangsrichtung beabstandeter digitaler Kameras (11), die Längsmittelachse (L_{A}) jeweils eines der Kunststoffspritzgussteile (2) in einer auf der Prüfspur (13) liegenden Prüfposition (23), bevorzugt senkrecht, schneidet/schneiden.

3. Verpackungsverfahren nach einem der Ansprüche 1 oder 2,
dass die Transfermittel (14) einen ersten und einen zweiten Werkstückträger (15, 16) zur jeweiligen Aufnahme (5) einer der S Subgruppen (10) umfassen, wobei die Werkstückträger (15, 16) entlang einer Transferachse (T_{A}) zwischen einer jeweiligen Beladeposition, in der sie mit einer der S Subgruppen (10) beladen werden und einer jeweiligen Entladeposition, aus der die jeweilige Subgruppe (10) nach der optischen Prüfung in die zweite Ablageebene (7) überführt wird hin- und her verstellt werden, und dass der erste und der zweite Werkstückträger (15, 16) während des Transfers von der jeweiligen Beladeposition zur jeweiligen Entladeposition mit einer der S Subgruppen (10) entlang einer gemeinsamen Prüfspur (13) durch die Seitenprüfeinrichtung (9) gefördert werden.

4. Verpackungsverfahren nach Anspruch 3,
**dadurch gekennzeichnet,**
**dass** die, bevorzugt einen Riementrieb (17) umfassenden, Transfermittel (14) die Werkstückträger (15, 16) in einer elektronisch oder mechanisch gekoppelten Bewegung zwischen der jeweiligen Belade- und Entnahmeposition hin- und her verstellen.

5. Verpackungsverfahren nach einem der Ansprüche 3 oder 4,
**dadurch gekennzeichnet,**
**dass** zumindest einer der Werkstückträger (15, 16), bevorzugt beide Werkstückträger (15, 16), mit Verstellmitteln (27) relativ, insbesondere senkrecht, zu der Transferachse T_{A} verstellt wird/werden, derart, dass die Werkstückträger (15, 16) in der Seitenrüfeinrichtung (9) aneinander vorbeiverstellt werden, während sich ein mit einer der S Subgruppen (10) befüllter Werkstückträger (15, 16) der Werkstückträger (15, 16) in der Prüfspur (13) befindet.

6. Verpackungsverfahren nach einem der Ansprüche 3 oder 4,
**dadurch gekennzeichnet,**
**dass** die Werkstückträger (15, 16) jeweils eine Seitenöffnung (19, 20) aufweisen, in die die Kunststoffspritzgussteile (2) einer in dem jeweiligen Werkstückträger (15, 16) angeordneten Subgruppe (10) hineinragen, bevorzugt diese vertikal durchsetzen, und von der mindestens einen digitalen Kamera (11) durch die Seitenöffnung (19, 20) hindurch optisch geprüft werden.

7. Verpackungsverfahren nach Anspruch 6,
**dadurch gekennzeichnet,**
**dass** mindestens eine Kamera (11) der Seitenprüfeinrichtung (9) derart angeordnet ist, dass die Kunststoffspritzgussteile (2) im ersten Werkstückträger (15) durch die Seitenöffnung (19, 20) des zweiten Werkstückträgers (16) hindurch optisch geprüft werden und/oder derart, dass die Kunststoffspritzgussteile (2) im zweiten Werkstückträger (16) durch die Seitenöffnung (19, 20). des ersten Werkstückträgers (15) hindurch optisch geprüft werden.

8. Verpackungsverfahren nach einem der vorhergehenden Ansprüche, **dadurch gekennzeichnet,**
**dass** Kunststoffspritzgussteile (2) mit während der optischen Prüfung festgestellten Fehlern aussortiert und durch fehlerfreie Kunststoffspritzgussteile (2) aus der jeweils selben Kavität ersetzt werden oder eine Untergruppe mit fehlerhaftem Kunsstoffspritzgussteil (2) vollständig aussorteirt wird.

9. Verpackungsvorrichtung (1) zum Verpacken von als Pipettenspitzen oder medizinische Reaktionsgefäße ausgebildeten Kunststoffspritzgussteilen (2) in Endverpackungseinheiten, ausgebildet zur Durchführung eines Verpackungsverfahrens nach einem der vorhergehenden Ansprüche, und zum Zusammenwirken einer benachbart zu der Verpackungsvorrichtung (1) anordnenbaren Spritzgussvorrichtung, die ausgebildet ist zum Herstellen von Pipettenspitzen oder medizinischen Reaktionsgefäßen umfassend:
- mindestens einen Entnahmegreifer, der ausgebildet und von Steuermitteln angesteuert ist zum mehrfachen, jeweils gleichzeitigen Entnehmen eines jeweiligen Schusses von K Kunststoffspritzgussteilen (2) aus K Kavitäten einer Spritzgussvorrichtung, bei der bevorzugt die K Kavitäten, insbesondere gleichmäßig, auf C Cluster verteilt angeordnet sind,
- Untergruppenbildungsmittel, die ausgebildet und von Steuermitteln angesteuert sind zum gleichmäßigen und kavitätenreinen Verteilen der K Kunststoffspritzgussteile (2) auf U kavitätenreine Untergruppen (6) in einer ersten Ablageebene (4), bevorzugt bis jede Untergruppe L Kunststoffspritzgussteile (2) aufweist, wobei die Anzahl L der Kunststoffspritzgussteile (2) einer Untergruppe der Anzahl A der in einer Endverpackungseinheit maximal aufnehmbaren Kunststoffspritzgussteilen (2) oder einem ganzzahligen Teiler von A entspricht,
- Mittel, die ausgebildet sind zum kavitätenreinen und/oder kavitätensortierten Auffüllen der Endverpackungseinheiten, bis die Endverpackungseinheiten jeweils A Kunststoffspritzgussteile (2) aufweisen,
**dadurch gekennzeichnet,**
**dass** die Verpackungsvorrichtung (1) eine, mindestens eine, bevorzugt mehrere in einer Umfangsrichtung beabstandete, digitale Kamera/s (11) umfassende Seitenprüfeinrichtung (9) aufweist, die ausgebildet und angesteuert ist, um die Kunststoffspritzgussteile (2) der Untergruppen (6) einer optischen Seitenprüfung, insbesondere auf Form- und/oder Oberflächenfehler, vor dem Auffüllen der Endverpackungen zu, und
**dass** die Verpackungseinrichtung (1) erste Subgruppengreifer zum Aufteilen der Untergruppen (6) in jeweils S Subgruppen (10) mit, insbesondere linienförmig, hintereinander angeordneten Kunststoffspritzgussteilen (2) und zum nacheinander Überführen der S Subgruppen (10) Transfermittel (14) aufweist, mit denen die S Subgruppen (10) nacheinander durch die Seitenprüfeinrichtung (9) zur optischen Seitenprüfung förderbar sind, und
**dass** die Verpackungsvorrichtung (1) zweite Subgruppengreifer umfasst, die derart ausgebildet und von Steuermitteln angesteuert sind, dass die S Subgruppen (10) nach Durchlaufen der Seitenprüfeinrichtung (9) in einer zweiten Ablageebene (7) zu geprüften kavitätenreinen Untergruppen angeordnet werden, wobei deren Zusammensetzung für den Fall der Nicht-Aussortierung einzelner Kunststoffspritzgussteile aufgrund einer Fehlererkennung bevorzugt der Zusammensetzung der Untergruppen (6) der ersten Ablageebene (4) entspricht, und dass die zweite Ablageebene (7) entweder unmittelbar von den Endverpackungseinheiten oder von einem statischen oder verstellbaren, bevorzugt horizontalen, Zwischenspeicher gebildet ist und die geprüften Untergruppen aus dem Zwischenspeicher in die Endverpackungseinheiten mit Greifermitteln überführbar sind.

10. Verpackungsvorrichtung nach Anspruch 9,
**dadurch gekennzeichnet,**
**dass** eine optische Achse (K_{A}) der mindestens einen digitalen Kamera (11), bevorzugt die optischen Achsen (K_{A}) mehrerer in Umfangsrichtung beabstandeter Kameras (11), der Prüfeinrichtung winklig, insbesondere senkrecht, zur einer Vertikalachse der Prüfvorrichtung angeordnet ist, wobei vorzugsweise die Vertikalachse eine auf der Prüfspur (13) liegende Prüfposition (23) für die Kunststoffspritzgussteile (2) durchsetzt.

11. Verpackungsvorrichtung nach Anspruch 9 oder 10,
**dadurch gekennzeichnet,**
**dass** die Verpackungsvorrichtung (1) Transfermittel (14) zum Fördern der S Subgruppen (10) entlang einer gemeinsamen Prüfspur (13) durch die Seitenprüfeinrichtung (9) aufweist, und dass die Transfermittel (14) einen ersten und einen zweiten Werkstückträger (15, 16) zur jeweiligen Aufnahme (5) einer der S Subgruppen (10) umfassen, wobei die Werkstückträger (15, 16) entlang einer Transferachse zwischen einer jeweiligen Beladeposition, in der sie mit einer der S Subgruppen (10) beladbar sind und einer jeweiligen Entladeposition, aus der die jeweilige Subgruppe (10) nach der optischen Prüfung in die zweite Ablageebene (7) überführbar ist hin- und herverstellbar sind.

12. Verpackungsvorrichtung nach Anspruch 11,
**dadurch gekennzeichnet,**
**dass** zumindest einem der Werkstückträger (15, 16), bevorzugt den Werkstückträgern (15, 16), insbesondere pneumatische oder servomotorische, Verstellmittel (27) zugeordnet sind, mit denen zumindest einer der Werkstückträger (15, 16), bevorzugt die Werkstückträger, (15, 16) derart relativ, insbesondere senkrecht, zu der Transferachse (T_{A}) derart verstellbar ist/sind, dass die Werkstückträger (15, 16) in der Prüfeinrichtung kollisionsfrei aneinander vorbeibewegbar sind, während sich einer der Werkstückträger (15, 16) in der Prüfspur (13) befindet.

13. Verpackungsvorrichtung nach einem der Ansprüche 12 oder 13,
**dadurch gekennzeichnet,**
**dass** die, bevorzugt einen Riementrieb (17) umfassenden, Transfermittel (14) die Werkstückträger (15, 16) in einer elektronisch oder mechanisch gekoppelten Bewegung zwischen der jeweiligen Belade- und Entnahmeposition hin- und her verstellend ausgebildet sind.

14. Verpackungsvorrichtung nach einem der Ansprüche 11 bis 13 soweit rückbezogen auf Anspruch 10,
**dadurch gekennzeichnet,**
**dass** die Werkstückträger (15, 16) seitliche Öffnungen (19, 20) zur Aufnahme (5) der Kunststoffspritzgussteile (2) einer Subgruppe (10) aufweisen, wobei die Öffnungen (19, 20) derart angeordnet sind, dass diese beim Aneinander-Vorbeitransport der Werkstückträger (15, 16) in der Seitenprüfeinrichtung (9) von der optischen Achse (K_{A}) der mindestens einen digitalen Kamera (11), bevorzugt von mehreren optischen Achsen mehrerer in Umfangsrichtung beabstandeter Kameras (11) durchsetzt sind.

15. Spritzgusssystem, umfassend eine Spritzgussvorrichtung, die ausgebildet ist zum Herstellen von Pipettenspitzen oder medizinischen Reaktionsgefäßen, mit K Kavitäten, die bevorzugt, insbesondere gleichmäßig, auf C Cluster verteilt angeordnet sind sowie eine Verpackungsvorrichtung (1) nach einem der Ansprüche 9 bis 14.

## Claims

1. A packaging method for packaging injection-molded plastic parts (2) in final package units, the injection-molded plastic parts (2) being pipette tips or medical reaction vessels and each having a longitudinal center axis (L_{A}), which preferably extends centrally through an opening (3), the method comprising the steps of:
- simultaneously removing multiple shots of K injection-molded plastic parts (2) from K respective cavities, the K cavities preferably being distributed, in particular evenly, over C clusters,
- evenly distributing the K injection-molded plastic parts (2) in a cavity-pure manner over U cavity-pure subgroups (6) in a first storing plane (4), preferably until each subgroup contains L injection-molded plastic parts (2), the number L of the injection-molded plastic parts (2) of one subgroup (6) corresponding to the maximum number A of injection-molded plastic parts (2) that can be contained in one final package unit or to an integer divisor of A,
- filling up the final package units in a cavity-pure or cavity-sorted manner until the final package units each contain A injection-molded plastic parts (2),
**characterized in that**
the injection-molded plastic parts (2) of the subgroups (6) are subjected to an optical lateral inspection, in particular for shape and/or surface defects, in a lateral inspection device (9), which comprises at least one digital camera (11), preferably multiple digital cameras (11) spaced apart in a circumferential direction, before the final packages are filled up, and
for this purpose, the subgroups (6) are each divided into S sub-subgroups (10) of injection-molded plastic parts (2) disposed one behind the other, in particular linearly, and the S sub-subgroups (10) are transported through the lateral inspection device (9) one after the other by means of transfer means (14) and are subjected to the lateral inspection there, and
the S sub-subgroups (10) are arranged in inspected cavity-pure subgroups in a second storing plane (7) after having passed through the lateral inspection device (9), their composition preferably corresponding to the composition of the subgroups (6) of the first storing plane (4) in the event that no individual injection-molded plastic parts have been sorted out because of defects detected, and the second storing plane (7) being formed either directly by the final package units or by a stationary or adjustable, preferably horizontal, intermediate storage, and the inspected subgroups being moved from the intermediate storage into the final package units.

2. The packaging method according to claim 1,
**characterized in that**
the sub-subgroups (10) are transported through the lateral inspection device (9) along an inspection track (13) by means of the transfer means (14), in particular in such a manner that an optical axis of the at least one camera (11), preferably the optical axes of multiple digital cameras (11) spaced apart in the circumferential direction, each intersect(s), preferably perpendicularly, the longitudinal center axis (L_{A}) of one of the injection-molded plastic parts (2) in an inspection position (23) located on the inspection track (13).

3. The packaging method according to any one of claims 1 or 2,
**characterized in that**
the transfer means (14) comprise a first and a second work piece support (15, 16) each serving to receive one of the S sub-subgroups (10), the work piece supports (15, 16) being moved back and forth along a transfer axis (T_{A}) between respective loading positions, in which they are loaded with one of the S sub-subgroups (10), and respective unloading positions, from which the respective sub-subgroups (10) are transferred into the second storing plane (7) after optical inspection, and that the first and the second work piece support (15, 16) are conveyed through the lateral inspection device (9) along a shared inspection track (13) with one of the S sub-subgroups (10) while being transferred from the respective loading positions to the respective unloading positions.

4. The packaging method according to claim 3,
**characterized in that**
the transfer means (14), which preferably comprise a belt drive (17), move the work piece supports (15, 16) back and forth between the respective loading and unloading positions in an electronically or mechanically coupled movement.

5. The packaging method according to any one of claims 3 or 4,
**characterized in that**
at least one of the work piece supports (15, 16), preferably both work piece support (15, 16), is/are moved relative, in particular perpendicular, to the transfer axis Ta, by means of moving means (27) in such a manner that the work piece supports (15, 16) are moved past each other in the lateral inspection device (9) while one work piece support (15, 16) of the work piece supports (15, 16) which is filled with one of the S sub-subgroups (10) is located on the inspection track (13).

6. The packaging method according to any one of claims 3 or 4, **characterized in that**
the work piece supports (15, 16) each have a lateral opening (19, 20) into which the injection-molded plastic parts (2) of a sub-subgroup (10) disposed in the work piece support (15, 16) in question protrude, the injection-molded plastic parts (2) preferably vertically extending through the lateral opening (19, 20), and the work piece supports (15, 16) are optically inspected by the at least one digital camera (11) through the lateral opening (19, 20).

7. The packaging method according to claim 6,
**characterized in that**
at least one camera (11) of the lateral inspection device (9) is disposed in such a manner that the injection-molded plastic parts (2) in the first work piece support (15) are optically inspected through the lateral opening (19, 20) of the second work piece support (16) and/or in such a manner that the injection-molded plastic parts (2) in the second work piece support (16) are optically inspected through the lateral opening (19, 20) of the first work piece support (15).

8. The packaging method according to any one of the preceding claims,
**characterized in that**
injection-molded plastic parts (2) with defects detected during the optical inspection are sorted out and replaced with non-defective injection-molded plastic parts (2) from the same cavity, or a subgroup with a defective injection-molded plastic part (2) is sorted out entirely.

9. A packaging device (1) for packaging injection-molded plastic parts (2) in final package units, the injection-molded plastic parts (2) being pipette tips or medical reaction vessels, the packaging device (1) being configured to implement a packaging method according to any one of the preceding claims and to interact with an injection molding device disposed adjacent to the packaging device (1) and configured to produce pipette tips or medical reaction vessels, the packaging device (1) comprising:
- at least one removing gripper configured and controlled by control means to simultaneously remove multiple shots of K injection-molded plastic parts (2) from K respective cavities of an injection molding device, the K cavities preferably being distributed, in particular evenly, over C clusters,
- subgroup forming means configured and controlled by control means to evenly distribute the K injection-molded plastic parts (2) in a cavity-pure manner over U cavity-pure subgroups (6) in a first storing plane (4), preferably until each subgroup contains L injection-molded plastic parts (2), the number L of the injection-molded plastic parts (2) of one subgroup corresponding to the maximum number A of injection-molded plastic parts (2) that can be contained in one final package unit or to an integer divisor of A,
- means configured to fill up the final package units in a cavity-pure and/or cavity-sorted manner until the final package units each contain A injection-molded plastic parts (2),
**characterized in that**
the packaging device (1) has a lateral inspection device (9) comprising at least one digital camera (11), preferably multiple digital cameras (11) spaced apart in a circumferential direction, the lateral inspection device (9) being configured and controlled to subject the injection-molded plastic parts (2) of the subgroups (6) to an optical lateral inspection, in particular for shape and/or surface defects, before the final packages are filled up, and
the packaging device (1) has first sub-subgroup grippers for dividing each of the subgroups (6) into S sub-subgroups (10) of injection-molded plastic parts (2) disposed one behind the other, in particular linearly, and transfer means (14) for transferring the S sub-subgroups (10) one after the other, the transfer means (14) being configured to transport the S sub-subgroups (10) through the lateral inspection device (9) one after the other for optical lateral inspection, and
the packaging device (1) has second sub-subgroup grippers configured and controlled by control means in such a manner that the S sub-subgroups (10) are arranged in inspected cavity-pure subgroups in a second storing plane (7) after having passed through the lateral inspection device (9), their composition preferably corresponding to the composition of the subgroups (6) of the first storing plane (4) in the event that no individual injection-molded plastic parts have been sorted out because of defects detected, and that the second storing plane (7) is formed either directly by the final package units or by a stationary or adjustable, preferably horizontal, intermediate storage, and the inspected subgroups are movable from the intermediate storage into the final package units by gripper means.

10. The packaging device according to claim 9,
**characterized in that**
an optical axis (K_{A}) of the at least one digital camera (11) of the inspection device, preferably the optical axes (K_{A}) of multiple cameras (11) spaced apart in the circumferential direction, is/are disposed at an angle, in particular perpendicular, to a vertical axis of the inspection device, the vertical axis preferably running through an inspection position (23) for the injection-molded plastic parts (2) which is located on the inspection track (13).

11. The packaging device according to claim 9 or 10,
**characterized in that**
the packaging device (1) has transfer means (14) for conveying the S sub-subgroups (10) through the lateral inspection device (9) along a shared inspection track (13), and the transfer means (14) comprise a first and a second work piece support (15, 16) each serving to receive one of the S sub-subgroups (10), the work piece supports (15, 16) being configured to be moved back and forth along a transfer axis between respective loading positions, in which they are loaded with one of the S sub-subgroups (10), and respective unloading positions, from which the respective sub-subgroups (10) are transferred into the second storing plane (7) after optical inspection.

12. The packaging device according to claim 11,
**characterized in that**
moving means (27), in particular pneumatic or servomotor moving means, are assigned to at least one of the work piece supports (15, 16), preferably to the work piece supports (15, 16), the moving means (27) being configured to move at least one of the work piece supports (15, 16), preferably the work piece supports, (15, 16), relative, in particular perpendicular, to the transfer axis (T_{A}) in such a manner that the work piece supports (15, 16) can be moved past each other without collision in the inspection device while one of the work piece supports (15, 16) is located on the inspection track (13).

13. The packaging device according to any one of claims 12 or 13,
**characterized in that**
the transfer means (14), which preferably comprise a belt drive (17), are configured to move the work piece supports (15, 16) back and forth between the respective loading and unloading positions in an electronically or mechanically coupled movement.

14. The packaging device according to any one of claims 11 to 13 in terms of their dependence on claim 10,
**characterized in that**
the work piece supports (15, 16) have lateral openings (19, 20) for receiving the injection-molded plastic parts (2) of a sub-subgroup (10), the openings (19, 20) being disposed in such a manner that they are traversed by the optical axis (K_{A}) of the at least one digital camera (11), preferably by multiple optical axes of multiple cameras (11) spaced apart in the circumferential direction, when the work piece supports (15, 16) are transported past each other in the lateral inspection device (9).

15. An injection molding system comprising an injection molding device configured to produce pipette tips or medical reaction vessels, with K cavities, which are preferably distributed, in particular evenly, over C clusters, and a packaging device (1) according to any one of claims 9 to 14.

## Revendications

1. Procédé d'emballage de pièces (2) en matière plastique moulées par injection dans des unités d'emballage final, les pièces (2) en matière plastique moulées par injection étant des pointes de pipettes ou des récipients de réaction médicaux et ayant chacune un axe central longitudinal (L_{A}), qui s'étend de préférence centralement à travers une ouverture (3), le procédé comprenant les étapes consistant à :
- enlever simultanément plusieurs tirs de K pièces (2) en matière plastique moulées par injection de K cavités respectives, les K cavités étant de préférence reparties, notamment uniformément, sur C clusters,
- repartir uniformément les K pièces (2) en matière plastique moulées par injection de manière fidèle à la cavité sur U sous-groupes (6) fidèles à la cavité dans un premier plan de stockage (4), de préférence jusqu'à ce que chaque sous-groupe contienne L pièces (2) en matière plastique moulées par injection, le nombre L des pièces (2) en matière plastique moulées par injection d'un sous-groupe (6) correspondant au nombre A maximal de pièces (2) en matière plastique moulées par injection qui peuvent être contenues dans une unité d'emballage final ou à un diviseur entier d'A,
- remplir les unités d'emballage final de manière fidèle à la cavité ou triée par cavité jusqu'à ce que les unités d'emballage final contiennent chacune A pièces (2) en matière plastique moulées par injection,
**caractérisé en ce que**
les pièces (2) en matière plastique moulées par injection des sous-groupes (6) sont soumises à une inspection latérale optique, notamment pour vérifier l'absence de défauts de forme et/ou de surface, dans un dispositif d'inspection latérale (9), qui comprend au moins un appareil photo numérique (11), de préférence plusieurs appareils photo numériques (11) espacés dans une direction circonférentielle, avant le remplissage des emballages finaux, et
à cette fin, les sous-groupes (6) sont divisés chacun en S sous-sous-groupes (10) de pièces (2) en matière plastique moulées par injection disposées l'une derrière l'autre, notamment linéairement, et les S sous-sous-groupes (10) sont transportés à travers le dispositif d'inspection latérale (9) l'un après l'autre par des moyens de transfert (14), où il sont soumis à l'inspection latérale, et
les S sous-sous-groupes (10) sont disposés dans des sous-groupes fidèles à la cavité inspectés dans un deuxième plan de stockage (7) après avoir traversé le dispositif d'inspection latérale (9), leur composition correspondant de préférence à la composition des sous-groupes (6) du premier plan de stockage (4) au cas où aucune pièce en matière plastique moulée par injection individuelle n'a été triée en raison de défauts détectés, et le deuxième plan de stockage (7) étant formé soit directement par les unités d'emballage final soit par un stockage intermédiaire fixe ou ajustable, de préférence horizontal, et les sous-groupes inspectés étant transférés du stockage intermédiaire dans les unités d'emballage final.

2. Procédé d'emballage selon la revendication 1,
**caractérisé en ce que**
les sous-sous-groupes (10) sont transportés à travers le dispositif d'inspection latérale (9) le long d'une piste d'inspection (13) par les moyens de transfert (14), notamment de telle manière qu'un axe optique de l'au moins un appareil photo (11), de préférence les axes optiques de plusieurs appareils photo numériques (11) espacés dans la direction circonférentielle, croise(nt) chacun, de préférence perpendiculairement, l'axe central longitudinal (L_{A}) d'une des pièces (2) en matière plastique moulées par injection dans une position d'inspection (23) située sur la piste d'inspection (13).

3. Procédé d'emballage selon l'une quelconque des revendications 1 ou 2,
**caractérisé en ce que**
les moyens de transfert (14) comprennent un premier et un deuxième support d'ouvrage (15, 16) servant chacun à recevoir un des S sous-sous-groupes (10), les supports d'ouvrage (15, 16) étant déplacés en va-et-vient le long d'un axe de transfert (T_{A}) entre des positions de chargement respectives, dans lesquelles ils sont chargés d'un des S sous-sous-groupes (10), et des positions de déchargement respectives, desquelles les sous-sous-groupes (10) respectives sont transférés dans le deuxième plan de stockage (7) après l'inspection optique, et que le premier et le deuxième support d'ouvrage (15, 16) sont transportés à travers le dispositif d'inspection latérale (9) le long d'une piste d'inspection (13) commune avec un des S sous-sous-groupes (10) lorsqu'ils sont transférés des positions de chargement respectives à les positions de déchargement respectives.

4. Procédé d'emballage selon la revendication 3,
**caractérisé en ce que**
les moyens de transfert (14), qui comprennent de préférence un entraînement par courroie (17), déplacent les supports d'ouvrage (15, 16) en va-et-vient entre les positions de chargement et de déchargement respectives par un mouvement couplé électroniquement ou mécaniquement.

5. Procédé d'emballage selon l'une quelconque des revendications 3 ou 4,
**caractérisé en ce**
**qu'**au moins un des supports d'ouvrage (15, 16), de préférence les deux support d'ouvrage (15, 16), est/sont déplacé(s) par rapport, notamment perpendiculairement, à l'axe de transfert T_{A} par des moyens de déplacement (27) de telle manière que les supports d'ouvrage (15, 16) passent l'un devant l'autre dans le dispositif d'inspection latérale (9) tandis qu'un support d'ouvrage (15, 16) des supports d'ouvrage (15, 16) qui est rempli d'un des S sous-sous-groupes (10) se trouve sur la piste d'inspection (13).

6. Procédé d'emballage selon l'une quelconque des revendications 3 ou 4,
**caractérisé en ce que**
les supports d'ouvrage (15, 16) ont chacun une ouverture latérale (19, 20) dans laquelle les pièces (2) en matière plastique moulées par injection d'un sous-sous-groupe (10) disposé dans le support d'ouvrage (15, 16) en question font saillie, les pièces (2) en matière plastique moulées par injection s'étendant de préférence verticalement à travers l'ouverture latérale (19, 20), et les supports d'ouvrage (15, 16) sont inspectés optiquement par l'au moins un appareil photo numérique (11) à travers l'ouverture latérale (19, 20).

7. Procédé d'emballage selon la revendication 6,
**caractérisé en ce**
**qu'**au moins un appareil photo (11) du dispositif d'inspection latérale (9) est disposé de telle manière que les pièces (2) en matière plastique moulées par injection dans le premier support d'ouvrage (15) sont inspectées optiquement à travers l'ouverture latérale (19, 20) du deuxième support d'ouvrage (16) et/ou de telle manière que les pièces (2) en matière plastique moulées par injection dans le deuxième support d'ouvrage (16) sont inspectées optiquement à travers l'ouverture latérale (19, 20) du premier support d'ouvrage (15).

8. Procédé d'emballage selon l'une quelconque des revendications précédentes,
**caractérisé en ce que**
des pièces (2) en matière plastique moulées par injection ayant des défauts détectés durant l'inspection optique sont triées et remplacées par des pièces (2) en matière plastique moulées par injection non défectueuses de la même cavité, ou un sous-groupe contenant une pièce en matière plastique moulée par injection (2) défectueuse est trié entièrement.

9. Dispositif (1) d'emballage de pièces (2) en matière plastique moulées par injection dans des unités d'emballage final, les pièces (2) en matière plastique moulées par injection étant des pointes de pipettes ou des récipients de réaction médicaux, le dispositif (1) d'emballage étant configuré pour réaliser un procédé d'emballage selon l'une quelconque des revendications précédentes et pour interagir avec un dispositif de moulage par injection disposé adjacent au dispositif (1) d'emballage et configuré pour produire des pointes de pipettes ou des récipients de réaction médicaux, le dispositif (1) d'emballage comprenant :
- au moins une griffe d'enlèvement configurée et actionnée par des moyens de commande pour enlever simultanément plusieurs tirs de K pièces (2) en matière plastique moulées par injection de K cavités respectives d'un dispositif de moulage par injection, les K cavités étant de préférence reparties, notamment uniformément, sur C clusters,
- des moyens de formation de sous-groupes configurés et actionnés par des moyens de commande pour repartir uniformément les K pièces (2) en matière plastique moulées par injection de manière fidèle à la cavité sur U sous-groupes (6) fidèles à la cavité dans un premier plan de stockage (4), de préférence jusqu'à ce que chaque sous-groupe contienne L pièces (2) en matière plastique moulées par injection, le nombre L des pièces (2) en matière plastique moulées par injection d'un sous-groupe correspondant au nombre A maximal de pièces (2) en matière plastique moulées par injection qui peuvent être contenues dans une unité d'emballage final ou à un diviseur entier d'A,
- des moyens configurés pour remplir les unités d'emballage final de manière fidèle à la cavité et/ou triée par cavité jusqu'à ce que les unités d'emballage final contiennent chacune A pièces (2) en matière plastique moulées par injection,
**caractérisé en ce que**
le dispositif (1) d'emballage a un dispositif d'inspection latérale (9) comprenant au moins un appareil photo numérique (11), de préférence plusieurs appareils photo numériques (11) espacés dans une direction circonférentielle, le dispositif d'inspection latérale (9) étant configuré et actionné pour soumettre les pièces (2) en matière plastique moulées par injection des sous-groupes (6) à une inspection latérale optique, notamment pour vérifier l'absence de défauts de forme et/ou de surface, avant le remplissage des emballages finaux, et
le dispositif (1) d'emballage a des premières griffes de sous-sous-groupe pour diviser chacun des sous-groupes (6) en S sous-sous-groupes (10) de pièces (2) en matière plastique moulées par injection disposées l'une derrière l'autre, notamment linéairement, et des moyens de transfert (14) pour transférer les S sous-sous-groupes (10) l'un après l'autre, les moyens de transfert (14) étant configurés pour transporter les S sous-sous-groupes (10) à travers le dispositif d'inspection latérale (9) l'un après l'autre pour l'inspection latérale optique, et
le dispositif (1) d'emballage a des deuxièmes griffes de sous-sous-groupe configurées et actionnées par des moyens de commande de telle manière que les S sous-sous-groupes (10) sont disposés dans des sous-groupes fidèles à la cavité inspectés dans un deuxième plan de stockage (7) après avoir traversé le dispositif d'inspection latérale (9), leur composition correspondant de préférence à la composition des sous-groupes (6) du premier plan de stockage (4) au cas où aucune pièce en matière plastique moulée par injection individuelle n'a été triée en raison de défauts détectés, et que le deuxième plan de stockage (7) est formé soit directement par les unités d'emballage final soit par un stockage intermédiaire fixe ou ajustable, de préférence horizontal, et les sous-groupes inspectés peuvent être transférés du stockage intermédiaire dans les unités d'emballage final par des moyens de griffe.

10. Dispositif d'emballage selon la revendication 9,
**caractérisé en ce**
**qu'**un axe optique (K_{A}) de l'au moins un appareil photo numérique (11) du dispositif d'inspection, de préférence les axes optiques (K_{A}) de plusieurs appareils photo (11) espacés dans la direction circonférentielle, est/sont disposé(s) selon un angle, notamment perpendiculairement, à un axe vertical du dispositif d'inspection, l'axe vertical s'étendant de préférence à travers une position d'inspection (23) pour les pièces (2) en matière plastique moulées par injection qui est située sur la piste d'inspection (13).

11. Dispositif d'emballage selon la revendication 9 ou 10,
**caractérisé en ce que**
le dispositif (1) d'emballage a des moyens de transfert (14) pour transporter les S sous-sous-groupes (10) à travers le dispositif d'inspection latérale (9) le long d'une piste d'inspection (13) commune, et les moyens de transfert (14) comprennent un premier et un deuxième support d'ouvrage (15, 16) servant chacun à recevoir un des S sous-sous-groupes (10), les supports d'ouvrage (15, 16) étant configurés pour être déplacés en va-et-vient le long d'un axe de transfert entre des positions de chargement respectives, dans lesquelles ils sont chargés d'un des S sous-sous-groupes (10), et des positions de déchargement respectives, desquelles les sous-sous-groupes (10) respectives sont transférés dans le deuxième plan de stockage (7) après l'inspection optique.

12. Dispositif d'emballage selon la revendication 11,
**caractérisé en ce que**
des moyens de déplacement (27), notamment des moyens de déplacement pneumatiques ou servomoteurs, sont assignés à au moins un des supports d'ouvrage (15, 16), de préférence aux supports d'ouvrage (15, 16), les moyens de déplacement (27) étant configurés pour déplacer au moins un des supports d'ouvrage (15, 16), de préférence les supports d'ouvrage, (15, 16), par rapport, notamment perpendiculairement, à l'axe de transfert (T_{A}) de telle manière que les supports d'ouvrage (15, 16) peuvent passer l'un devant l'autre sans collision dans le dispositif d'inspection tandis qu'un des supports d'ouvrage (15, 16) se trouve sur la piste d'inspection (13).

13. Dispositif d'emballage selon l'une quelconque des revendications 12 ou 13,
**caractérisé en ce que**
les moyens de transfert (14), qui comprennent de préférence un entraînement à courroie (17), sont configurés pour déplacer les supports d'ouvrage (15, 16) en va-et-vient entre les positions de chargement et de déchargement respectives par un mouvement couplé électroniquement ou mécaniquement.

14. Dispositif d'emballage selon l'une quelconque des revendications 11 à 13 dans la mesure où elles dépendent de la revendication 10,
**caractérisé en ce que**
les supports d'ouvrage (15, 16) ont des ouvertures latérales (19, 20) pour recevoir les pièces (2) en matière plastique moulées par injection d'un sous-sous-groupe (10), les ouvertures (19, 20) étant disposées de telle manière qu'elles sont traversées par l'axe optique (K_{A}) de l'au moins un appareil photo numérique (11), de préférence par plusieurs axes optiques de plusieurs appareils photo (11) espacés dans la direction circonférentielle, lorsque les supports d'ouvrage (15, 16) passent l'un devant l'autre dans le dispositif d'inspection latérale (9).

15. Système de moulage par injection comprenant un dispositif de moulage par injection configuré pour produire des pointes de pipettes ou des récipients de réaction médicaux, avec K cavités, qui sont de préférence reparties, notamment uniformément, sur C clusters, et un dispositif (1) d'emballage selon l'une quelconque des revendications 9 à 14.
